(19)

Europäisches
Patentamt

European
Patent Office

Office européen
des brevets

(11) **EP 4 772 530 A1**

(12) **EUROPEAN PATENT APPLICATION**
published in accordance with Art. 153(4) EPC

(43) Date of publication:
**08.07.2026 Bulletin 2026/28**

(21) Application number: 23950151.3

(22) Date of filing: **30.08.2023**

(51) International Patent Classification (IPC):
*C07K 7/08* $^{(2006.01)}$　　*A61K 47/64* $^{(2017.01)}$
*A61K 31/7068* $^{(2006.01)}$　　*A61P 35/00* $^{(2006.01)}$

(52) Cooperative Patent Classification (CPC):
**A61K 31/7068; A61K 47/64; A61P 35/00;
C07K 7/08**

(86) International application number:
**PCT/CN2023/115938**

(87) International publication number:
**WO 2025/043563 (06.03.2025 Gazette 2025/10)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicants:
- **Mainline Biosciences (Shanghai) Co., Ltd.
  Shanghai 201422 (CN)**
- **Zhongshan Mainline Biosciences Co., Ltd.
  Zhongshan, Guangdong 528437 (CN)**

(72) Inventor: **ZHANG, Junge
Shanghai 201422 (CN)**

(74) Representative: **Cabinet Chaillot
16/20, avenue de l'Agent Sarre
B.P. 74
92703 Colombes Cedex (FR)**

Remarks:
The complete document including Reference
Table(s) and the Sequence Listing(s) can be
downloaded from the EPO website

(54) **PEPTIDE TARGETING SOMATOSTATIN RECEPTOR, DRUG CONJUGATE AND USE
THEREOF**

(57)　　Provided are a peptide targeting SSTR2, or a peptide specific for SSTR2. Further provided are a ligand, a drug conjugate, and use thereof. The peptide targeting SSTR2 has the following structure: Arg-X1-Phe-[Cys-Xaa-Trp-Lys-Thr-Cys]-Thr-X2-Arg-X3-NH₂/OH, wherein a disulfide bond is formed between the two Cys residues within the square brackets. The peptide/peptide ligand/peptide-drug conjugate provided herein can be used for diagnosing, preventing, inhibiting, or treating cancer, particularly solid tumors originating from neuroendocrine cells, such as neuroendocrine tumors of the digestive system such as the stomach, intestines, and pancreas, and small cell lung cancer.

**EP 4 772 530 A1**

Description

## FIELD OF THE INVENTION

The present disclosure relates to a novel peptide targeting SSTR2, in particular to a peptide-drug conjugate (PDC) comprising a peptide conjugated to one or more effectors and/or functional groups, a pharmaceutical composition comprising the peptide ligand and the PDC, and the use of the peptide ligand or the PDC in preventing, inhibiting, or treating diseases with high expression of SSTR2 or in related pharmaceuticals.

## BACKGROUND OF THE INVENTION

Somatostatin receptors (SSTRs) are a family of G protein-coupled receptors on the cell membrane that specifically bind to somatostatin. SSTR has been identified so far as having five different molecular subtypes, namely SSTR1 to SSTR5. Among them, SSTR2 has two distinct isomers (subunits), namely SSTR2A and SSTR2B. According to the degree of homology of amino acid sequence and the similarity of selectivity and functional response of related ligands, the SSTR family is divided into two large subgroups, one of which is composed of SSTR1 and 4, and the other of which is composed of SSTR2, 3 and 5.

At the 19th Annual ENETS Conference in 2022, research related to SSTR became a hot topic. The high heterogeneity and rarity of neuroendocrine tumors (NETs) pose significant challenges to precision oncology. In recent years, SSTR family targets have been recognized as the most important targets in NET treatment and also an important target in the diagnosis of NETs.

Among the SSTR family, the most extensively studied subtype is SSTR2. Clinically, it has been reported that over 70% of both small cell lung cancer (SCLC) and non-small cell lung cancer (NSCLC) cases exhibit SSTR expression, with SSTR2 predominantly expressed in SCLC and squamous cell carcinoma. A large body of data shows that SSTR2 is specifically expressed in NETs.

Reverse transcription polymerase chain reaction (RT-PCR) was used to detect SSTR2 and SSTR3 mRNA expression in tumor and adjacent tissues from 27 patients with primary liver cancer. Results: In the 27 patients with primary liver cancer, the positive rates of SSTR2 mRNA expression in tumor and adjacent tissues were 81.5%(22/27) and 96.3% (26/27), respectively. The positive rates of SSTR3 mRNA expression in tumor and adjacent tissues were 66.7%(18/27) and 51.9%(14/27), respectively. More than one SSTR subtype was expressed in the tumor tissues of most primary liver cancer cases.

The expression of SSTRs in the human nasopharyngeal carcinoma cell line CNE2 cultured *in vitro* was detected by RT-PCR and SP immunohistochemistry, and the PCR mRNA products were sequenced for identification. Results: RT-PCR revealed that CNE2 expressed SSTR1, SSTR2, and SSTR4; immunohistochemical results showed strong positivity for SSTR1 and SSTR2A (60%), weak positivity for SSTR4, moderate positivity for SSTR2, and an absence of expression for SSTR3 and SSTR5. Conclusion: The study demonstrates that CNE2 expresses multiple SSTR subtypes, especially SSTR1 and SSTR2.

Therefore, SSTR2 plays a crucial role as a therapeutic target in the treatment of NETs, offering a better strategy for NET therapy. Based on this, research on SSTR2-targeted drugs is of vital significance for the diagnosis, prevention, inhibition, or treatment of diseases with high expression of SSTR2.

## SUMMARY **OF THE INVENTION**

Provided are a peptide targeting SSTR2, or a peptide specific for SSTR2; further provided are a drug conjugate and use thereof.

In a first aspect of the present application, there is provided a peptide targeting SSTR2, which has the following structure: Cys-Xaa-Trp-Lys-Thr-Cys, wherein a disulfide bond is formed between the two Cys residues.

In a preferred embodiment, Xaa is selected from one or more of Tyr and Phe.

In a preferred embodiment, Trp is D-Trp.

In a preferred embodiment, the peptide targeting SSTR2 comprises the following sequence: Arg-X1-Phe-[Cys-Xaa-Trp-Lys-Thr-Cys]-Thr-X2-Arg-X3-$NH_2$/OH;

    wherein a disulfide bond is formed between the two Cys residues within the square brackets "[]";
    X1 and X2 may be any amino acid residue; X3 may be any amino acid residue or may be absent;
    wherein X1 is preferably Arg or Leu, X2 is preferably Trp or Asp, and X3 is preferably Asp.

In a preferred embodiment, the peptide targeting SSTR2 comprises the following sequence: X0-Arg-X1-Phe-[Cys-Xaa-Trp-Lys-Thr-Cys]-Thr-X2-Arg-X3-$NH_2$/OH;

wherein a disulfide bond is formed between the two Cys residues within the square brackets "[]";
wherein X0 may be an amino acid residue containing a group capable of reacting with an amino group or be another carboxyl-containing group, and the group capable of reacting with an amino group is preferably a carboxyl group. Examples of X0 include Arg or Gly.

In a second aspect of the present application, there is provided a peptide ligand targeting SSTR2, or a drug targeting SSTR2 containing the peptide ligand, comprising the peptide and a spacer conjugated to the peptide, wherein the spacer has the following structure: X4-X6-X5, wherein X4 may be absent or is a single bond, an acetyl group, or a polyether structure conjugated to the peptide, and X5 is a polyether structure, such as a structure with 2 to 12 repeating units of polyethylene glycol.

X6 may be selected from amino acid residues containing a reactive group such as carboxyl, hydroxyl, amino, thiol, and aldehyde; preferably, X6 may be selected from a group consisting of cysteine residues, lysine residues, ornithine residues, glutamic acid residues, aspartic acid residues, 6-azido-L-norleucine, 2-amino-5-hydroxypentanoic acid residues, 2-amino-5-ureidopentanoic acid residues, and 2-amino-5-cyanobenzoic acid residues.

For example, the spacer is X4-Cys-X5; wherein the spacer may further contain other amino acid residues, for example, the spacer may have the following structure: X4'-X6-{Lys(X4-X6'-X5)}x-AAy-X5', wherein AA is a random sequence of amino acid residues, y is an integer from 0 to 4, and x is an integer from 0 to 3, wherein X4' and X4 may be identical or different, and independently may be absent or is a single bond, an acetyl group, or a polyether structure conjugated to the peptide, such as a structure with 2 to 12 repeating units of polyethylene glycol; X5' and X5 may be identical or different, and each independently is a polyether structure, such as a structure with 2 to 12 repeating units of polyethylene glycol; X6 and X6' may be identical or different, and each independently is an amino acid residue containing a reactive group such as carboxyl, hydroxyl, amino, thiol, and aldehyde; preferably, X6 and X6' may each independently be selected from a group consisting of cysteine residues, lysine residues, ornithine residues, glutamic acid residues, aspartic acid residues, 6-azido-L-norleucine, 2-amino-5-hydroxypentanoic acid residues, 2-amino-5-ureidopentanoic acid residues, and 2-amino-5-cyanobenzoic acid residues.

The peptide ligand or drug described herein further comprises a linker for conjugating an active pharmaceutical ingredient, wherein the linker is conjugated to X6 and/or X6' of the spacer.

As a preferred example, the Cys-Xaa-Trp-Lys-Thr-Cys is selected from a group consisting of: Cys-Tyr-Trp-Lys-Thr-Cys and Cys-Phe-Trp-Lys-Thr-Cys.

As a preferred example, the peptide targeting SSTR2 is selected from the following sequences:

SEQ ID NO.1:
H-Arg-Arg-Leu-Phe-[Cys-Tyr-Trp-Lys-Thr-Cys]-Thr-Trp-Arg-Asp-NH$_2$
SEQ ID NO. 2:
H-Arg-Arg-Leu-Phe-[Cys-Phe-Trp-Lys-Thr-Cys]-Thr-Trp-Arg-Asp-NH$_2$
SEQ ID NO. 3:
H-Gly-Arg-Arg-Phe-[Cys-Tyr-Trp-Lys-Thr-Cys]-Thr-Asp-Arg-NH$_2$
SEQ ID NO. 4:
H-Arg-Arg-Leu-Phe-[Cys-Tyr-Trp-Lys-Thr-Cys]-Thr-Trp-Arg-Asp-OH

As a preferred example, the spacer is selected from a group consisting of: X6-(AEEA)r, PEGz-X6-PEGz, Ac-X6-PEGz, Ac-X6-(AEEA)r, Ac-X6-PEGz-{Lys(Ac-X6'-PEG6)}x-PEG6, and X6-Glu-Glu-Glu-AEEA; wherein r is an integer, such as an integer from 0 to 6, more preferably an integer from 1 to 5, such as 2, 3, and 4.

As a preferred example, the linker may be MC-Val-Ala-PABC, MC-Val-Cit-PABC, MC-Gly-Gly-Phe-Gly-NH-CH$_2$, MCC, MC-betaglucuronide, MC-Glu-Val-Ala-PABC, or MC-Glu-Val-Cit-PABC, wherein MC is maleimidocaproyl, PABC is p-aminobenzyl alcohol, Cit is citrulline, and MCC is 4-((2,5-dioxo-2,5-dihydro-1H-pyrrol-1-yl)methyl)cyclohexane-1-carboxylic acid.

The peptide-drug conjugate targeting SSTR2 described herein further comprises a active pharmaceutical ingredient (also referred to as an effector), which is conjugated to the linker; wherein the active pharmaceutical ingredient can be conjugated to the linker by means such as condensation, esterification, etherification, and amidation reaction.

As a preferred example, the active pharmaceutical ingredient may be a cytotoxic agent selected from a group consisting of: microtubule inhibitors (such as auristatins, maytansine derivatives, tubulysins, and cryptomycins), DNA-damaging drugs (such as pyrrolobenzodiazepines and indochloropyrrolobenzodiazepines, duocarmycin, camptothecin, and calicheamicin), amatoxins, apoptosis inducers, and camptothecins.

As a more preferred example, the cytotoxic agent is selected from a group consisting of MMAF, MMAE, PBD, DM1, DM4, Dxd, SN38, paclitaxel, docetaxel, and exatecan.

It should be noted, however, that the peptides described in the foregoing paragraphs of the present application may also be retro-inverso forms of the sequences described above. For example, the amino acid sequence is reversed (i.e., N-terminal to C-terminal and *vice versa),* and the stereochemistry is likewise reversed (i.e., D-amino acid to L-amino acid and *vice versa).* As disclosed in Nair et al.(*J* Immunol, 2003 170(3), pp. 1362-1373), these retro-inverso forms have the same or similar technical effects and are included within the scope of the peptides of the present application.

It should be noted, however, that the peptides or peptide ligands described in the foregoing paragraphs of the present application may also include all pharmaceutically acceptable (radioactive) isotope- or marker-labeled peptides or peptide ligands, or the active pharmaceutical ingredient is an isotope or marker. For example, one or more atoms are replaced by an atom having the same atomic number but an atomic mass or mass number different from that normally found in nature, or the payload is a metal chelating group capable of holding a (radioactive) isotope or marker, or certain functional groups in the peptide or peptide ligand are covalently substituted with related (radioactive) isotope- or marker-labeled functional groups.

Examples of isotopes suitable for inclusion in the present disclosure include hydrogen isotopes such as $^{2}$H and $^{3}$H, carbon isotopes such as $^{11}$C,$^{13}$C and $^{14}$C, chlorine isotopes such as $^{36}$Cl, fluorine isotopes such as $^{18}$F, iodine isotopes such as $^{123}$I,$^{125}$I and $^{131}$I, nitrogen isotopes such as $^{13}$N and $^{15}$N, oxygen isotopes such as $^{15}$O,$^{17}$O and $^{18}$O, phosphorus isotopes such as $^{32}$P, sulfur isotopes such as $^{35}$S, copper isotopes such as $^{64}$Cu, gallium isotopes such as $^{67}$Ga or $^{68}$Ga, yttrium isotopes such as $^{90}$Y, lutetium isotopes such as $^{177}$Lu, and bismuth isotopes such as $^{213}$Bi.

Markers suitable for inclusion in the present disclosure may be, for example, a radioactive isotope, an enzyme, a fluorescent substance, a luminescent substance (such as luminol, luminol derivatives, fluorescein,aequorin, and luciferase), and the like.

As a preferred example, the peptide-drug conjugate targeting SSTR2 is selected from:

Drug 1:

Cys(MC-Val-Cit-PABC-MMAE)-AEEA-Arg-Arg-Leu-Phe-[Cys-Tyr-D-Trp-Lys-Thr-Cys]-Thr-Trp-Arg-Asp-NH$_2$

Drug 2:

PEG$_6$-Cys(MC-Val-Cit-PABC-MMAE)-PEG$_6$-Arg-Arg-Leu-Phe-[Cys-Tyr-D-Trp-Lys-Thr-Cys]-Thr-Trp-Arg-Asp-NH$_2$

Drug 3:

Ac-Cys(MC-Val-Cit-PABC-MMAE)-PEG$_6$-Arg-Arg-Leu-Phe-[Cys-Tyr-D-Trp-Lys-Thr-Cys]-Thr-Trp-Arg-Asp-NH$_2$

Drug 4:

Ac-Cys(MC-betaglucuronide-MMAE)-AEEA-AEEA-AEEA-Arg-Arg-Leu-Phe-[Cys-Tyr-D-Trp-Lys-Thr-Cys]-Thr-Trp-Arg-Asp-NH$_2$

Drug 5:

PEG$_6$-Cys(MC-Gly-Gly-Phe-Gly-NH-CH$_2$-Dxd)-PEG$_6$-Arg-Arg-Leu-Phe-[Cys-Tyr-D-Trp-Lys-Thr-Cys]-Thr-Trp-Arg-Asp-NH$_2$

Drug 6:

Ac-Cys(MC-Gly-Gly-Phe-Gly-NH-CH$_2$-Dxd)-PEG$_6$-Arg-Arg-Leu-Phe-[Cys-Tyr-D-Trp-Lys-Thr-Cys]-Thr-Trp-Arg-Asp-NH$_2$

Drug 7:

Ac-Cys(MC-Gly-Gly-Phe-Gly-NH-CH2-Dxd)-PEG$_6$-Lys(Ac-Cys(MC-Gly-Gly-Phe-Gly-NH-CH$_2$-Dxd)-PEG$_6$)-Lys(Ac-Cys(MC-Gly-Gly-Phe-Gly-NH-CH2-Dxd)-PEG$_6$)-PEG6-Arg-Arg-Leu-Phe-[Cys-Tyr-D-Trp-Lys-Thr-Cys]-Thr-Trp-Arg-Asp-NH$_2$

Drug 8:

Ac-Cys(MC-Val-Cit-PABC-MMAE)-AEEA-AEEA-AEEA-Arg-Arg-Leu-Phe-[Cys-Phe-D-Trp-Lys-Thr-Cys]-Thr-Trp-Arg-Asp-NH$_2$

Drug 9:

Cys(MC-Val-Cit-PABC-MMAE)-Glu-Glu-Glu-AEEA-Arg-Arg-Leu-Phe-[Cys-Tyr    D-Trp-Lys-Thr-Cys]-Thr-Trp-Arg-Asp-NH$_2$

Drug 10:

Ac-Cys(MC-Val-Cit-PABC-MMAE)-AEEA-Arg-Arg-Leu-Phe-[Cys-Tyr-D-Trp-Lys-Thr-Cys]-Thr-Trp-Arg-Asp-NH$_2$

In a third aspect of the present application, there is provided the use of a peptide, peptide ligand, or peptide-drug conjugate targeting SSTR2 as described above for preventing, inhibiting, or treating a disease with high expression of SSTR2, or for preparing a medicament for treating, inhibiting, or preventing a disease with high expression of SSTR2;

wherein the peptide, peptide ligand, or peptide-drug conjugate is used for diagnosing, preventing, inhibiting, or treating cancer, or for preparing a medicament for diagnosing, preventing, inhibiting, or treating cancer;
wherein the cancer is particularly preferably solid tumors originating from neuroendocrine cells, such as neuroendocrine tumors of the digestive system such as the stomach, intestines, and pancreas, and small cell lung cancer.

The peptide, peptide ligand, and peptide-drug conjugate provided in the present disclosure target SSTR2 and can be used for diagnosing, preventing, inhibiting, or treating cancer,particularly solid tumors originating from neuroendocrine cells, such as neuroendocrine tumors of the digestive system such as the stomach, intestines, and pancreas, and small cell lung cancer.

## DETAILED DESCRIPTION

The present application provides a peptide and a peptide ligand targeting SSTR2, and further provides a peptide-drug conjugate targeting SSTR2 for use in diagnosing, preventing, inhibiting, or treating diseases with high expression of SSTR2.

In one embodiment, the peptide comprises the following sequence: Cys-Tyr/Phe-Trp-Lys-Thr-Cys, wherein, preferably, a disulfide bond is formed between the two Cys residues.

In a further preferred embodiment, the peptide comprises the following sequence:
Arg-X1-Phe-[Cys-Tyr/Phe-Trp-Lys-Thr-Cys]-Thr-X2-Arg-X3-NH$_2$/OH
or the peptide comprises the following sequence:

X0-Arg-X1-Phe-[Cys-Xaa-Trp-Lys-Thr-Cys]-Thr-X2-Arg-X3-NH$_2$/OH
wherein a disulfide bond is formed between the two Cys residues within the square brackets;
wherein X1 and X2 may be any amino acid residue, for example, X1 is Arg or Leu, and X2 is Trp or Asp; X3 may be any amino acid residue or may be absent, such as Asp;
wherein X0 may be an amino acid residue containing a group capable of reacting with an amino group or be another carboxyl-containing group, and the group capable of reacting with an amino group is preferably a carboxyl group. Examples of X0 include Arg or Gly.

Specifically, the peptide of the present application is selected from:

SEQ ID NO.1:
H-Arg-Arg-Leu-Phe-[Cys-Tyr-D-Trp-Lys-Thr-Cys]-Thr-Trp-Arg-Asp-NH$_2$
SEQ ID NO.2:
H-Arg-Arg-Leu-Phe-[Cys-Phe-D-Trp-Lys-Thr-Cys]-Thr-Trp-Arg-Asp-NH$_2$
SEQ ID NO.3:
H-Gly-Arg-Arg-Phe-[Cys-Tyr-D-Trp-Lys-Thr-Cys]-Thr-Asp-Arg-NH$_2$
SEQ ID NO.4:
H-Arg-Arg-Leu-Phe-[Cys-Tyr-D-Trp-Lys-Thr-Cys]-Thr-Trp-Arg-Asp-OH

In one embodiment, the peptide ligand further comprises a spacer, and may also comprise a linker.

Spacer

The functionalized spacer of the present disclosure is located at the N-terminal of the peptide and added to the left side of the peptide sequence. In one embodiment, the general formula of the functionalized spacer is X4-X6-X5, wherein X4 may preferably be a structure with 2 to 12 repeating units of polyethylene glycol, an acetyl group, or absent; X5 may preferably be a structure with 2 to 12 repeating units of polyethylene glycol; X6 is an amino acid residue containing a reactive group such as hydroxyl, carboxyl, amino, and thiol. For example, X6 may be selected from a group consisting of cysteine residues, lysine residues, ornithine residues, glutamic acid residues, aspartic acid residues, 6-azido-L-norleucine, 2-amino-5-hydroxypentanoic acid residues, 2-amino-5-ureidopentanoic acid residues, and 2-amino-5-cyanobenzoic acid residues.

Alternatively, the spacer may be X4'-X6-{Lys(X4-X6'-X5)}x-AAy-X5', wherein AA is a random sequence of amino acid residues, y is an integer from 0 to 4, and x is an integer from 0 to 3, wherein X4' and X4 may be identical or different, and independently may be absent or is a single bond, an acetyl group, or a polyether structure conjugated to the peptide, such as a structure with 2 to 12 repeating units of polyethylene glycol; X5' and X5 may be identical or different, and each independently is a polyether structure, such as a structure with 2 to 12 repeating units of polyethylene glycol; X6 and X6' are each an amino acid residue containing a reactive group such as hydroxyl, carboxyl, amino, and thiol. For example, X6 and X6' may be independently selected from a group consisting of cysteine residues, lysine residues, ornithine residues, glutamic acid residues, aspartic acid residues, 6-azido-L-norleucine, 2-amino-5-hydroxypentanoic acid residues, 2-amino-5-ureidopentanoic acid residues, and 2-amino-5-cyanobenzoic acid residues.

For example, the spacer may be preferably selected from a group consisting of: Ac-Cys-(AEEA)$_r$, PEG$_z$-Cys-PEG$_z$, Ac-Cys-PEG$_z$, Ac-Cys-(AEEA)$_r$, Ac-Cys-PEG$_6$-{Lys(Ac-Cys-PEG$_6$)}$_x$-PEG$_6$, Ac-Cys-Glu-Glu-Glu-AEEA, Ac-Lys-(AEEA)$_r$, PEG$_z$-Lys-PEG$_z$, Ac-Lys-PEG$_z$, Ac-Lys-(AEEA)$_r$, Ac-Lys-PEG$_6$-{Lys(Ac-Cys-PEG6)}$_x$-PEG$_6$, Ac-Cys-PEG$_6$-{Lys(Ac-Lys-PEG$_6$)}$_x$-PEG$_6$, Ac-Lys-Glu-Glu-Glu-AEEA, Ac-Clu-(AEEA)$_r$, PEG$_z$-Glu-PEG$_z$, Ac-Clu-PEG$_z$, Ac-Clu-(AEEA)$_r$, Ac-Clu-PEG$_6$-{Lys(Ac-Org-PEG6)}$_x$-PEG$_6$, Ac-Asn-Glu-Glu-Glu-AEEA, Ac-Org-PEG$_z$, and PEG$_z$-Asn-PEG$_z$.

Linker

One end of the linker is conjugated to the functional spacer (conjugated to X6 and/or X6' of the spacer) via a maleimidopropionic acid linker. In one embodiment, the linker is MC-Val-Ala-PABC, MC-Val-Cit-PABC, MC-Gly-Gly-Phe-Gly-NH-CH$_2$, MCC, MC-betaglucuronide, MC-Glu-Val-Ala-PABC, MC-Glu-Val-Cit-PABC, and the like.

In one embodiment, the peptide-drug conjugate further comprises, in addition to the peptide ligand, a active pharmaceutical ingredient (also referred to as an effector) to which the other end of the linker is conjugated.

Active pharmaceutical ingredient

The active pharmaceutical ingredient is conjugated to the linker through amide condensation, esterification, and the like. In one embodiment, the effector is a cytotoxic agent, such as microtubule inhibitors (such as auristatins, maytansine derivatives, tubulysins, and cryptomycins), DNA-damaging drugs (such as pyrrolobenzodiazepines and indochloropyrrolobenzodiazepines, duocarmycin, camptothecin, and calicheamicin), amatoxins, apoptosis inducers, and camptothecins.

In a particular further embodiment of the present disclosure, the cytotoxic agent used is MMAF, MMAE, PBD, DM1, DM4, Dxd, SN38, paclitaxel, docetaxel, exatecan, and the like.

In one embodiment, the peptide, peptide ligand, or peptide-drug conjugate of the present application may further contain a pharmaceutically acceptable (radioactive) isotope label or marker.

Labeling

One or more atoms are replaced by an atom having the same atomic number but an atomic mass or mass number different from that normally found in nature, or certain functional groups are covalently substituted with related (radioactive) isotopes or isotope-labeled functional groups, or the active pharmaceutical ingredient is a metal chelating group capable of holding a related (radioactive) isotope, or the peptide/peptide ligand is conjugated to a metal chelating group capable of holding a related (radioactive) isotope.

Examples of isotopes suitable for inclusion in the peptide ligand of the present disclosure include hydrogen isotopes such as $^{2}H$ and $^{3}H$, carbon isotopes such as $^{11}C$, $^{13}C$ and $^{14}C$, chlorine isotopes such as $^{36}Cl$, fluorine isotopes such as $^{18}F$, iodine isotopes such as $^{123}I$, $^{125}I$ and $^{131}I$, nitrogen isotopes such as $^{13}N$ and $^{15}N$, oxygen isotopes such as $^{15}O$, $^{17}O$ and $^{18}O$, phosphorus isotopes such as $^{32}P$, sulfur isotopes such as $^{35}S$, copper isotopes such as $^{64}Cu$, gallium isotopes such as $^{67}Ga$ or $^{68}Ga$, yttrium isotopes such as $^{90}Y$, lutetium isotopes such as $^{177}Lu$, and bismuth isotopes such as $^{213}Bi$.

Certain isotope-labeled peptides/peptide ligands/peptide-drug conjugates are useful for tissue distribution studies of drugs and/or substrates, and for clinically evaluating the presence and/or absence of SSTR2 targets on diseased tissues. The present application may further be used to detect or identify the formation of complexes between labeled compounds and other molecules, peptides, proteins, enzymes, or receptors. The detection or identification method may use a compound labeled with a marker, such as a radioactive isotope, an enzyme, a fluorescent substance, a luminescent substance (such as luminol, luminol derivatives, fluorescein, aequorin, and luciferase), and the like. The radioactive isotopes tritium (i.e., $^{3}H$) and carbon-14 (i.e., $^{14}C$) are particularly useful for this detection and identification purpose.

Substitution with heavier isotopes such as deuterium (i.e., $^{2}H$) may offer certain therapeutic advantages due to greater metabolic stability, such as increased *in vivo* half-life or reduced dosage requirements, and may therefore be preferred in some cases.

Substitution with positron emitting isotopes such as $^{11}C$, $^{18}F$, $^{15}O$, and $^{13}N$ can be used in positron emission imaging (PET) studies to examine target occupancy.

Labeling of the peptide/peptide ligand/peptide-drug conjugate of the present disclosure can generally be prepared by conventional techniques known to those skilled in the art or by methods analogous to those described in the accompanying Examples, using a suitable isotope-labeled reagent or maker in place of the previously employed non-labeled reagents.

## Example 1

## Synthesis of Cys(MC-Val-Cit-PABC-MMAE)-AEEA-Arg-Arg-Leu-Phe-[Cys-Tyr-D-Trp-Lys-Thr-Cys]-Thr-Trp-Arg-Asp-NH$_2$

Peptide synthesis was based on Fmoc chemistry using a solid-phase synthesis method and 0.2 mmol/g Rink Amide-AM Resin and standard Fmoc-amino acid. The coupling agents used were DIC and HOBt, and deprotection was carried out with 20% piperidine in DMF.

The resin modification was carried out using a 1.5-fold excess of Fmoc-Asp (OtBu)-OH and a 1.5-fold excess of coupling reagents. Fmoc-Arg(Pbf)-OH, Fmoc-Trp(Boc)-OH, Fmoc-Thr(tBu)-OH, Fmoc-Cys(Trt)-OH, Fmoc-Thr(tBu)-OH, Fmoc-Lys(Boc)-OH, Fmoc-D-Trp(Boc)-OH, Fmoc-Tyr(tBu)-OH, and Fmoc-Cys(Trt)-OH were sequentially condensed using a 3-fold excess of Fmoc-amino acid and a 3-fold excess of coupling reagents.

Solid phase cyclization was carried out with a 5-fold amount of iodine dissolved in DMF for 45 min, and after completion of the reaction, the resin was washed with $V_{DMF}$:$V_{ACN}$:$V_{H2O}$=90: 5: 5 and then washed three times with DMF.

Then, Fmoc-Phe-OH, Fmoc-Leu-OH, Fmoc-Arg(Pbf)-OH, Fmoc-Arg(Pbf)-OH, AEEA, and Fmoc-Cys(Trt)-OH were sequentially condensed using a 3-fold excess of Fmoc-amino acid and a 3-fold excess of coupling reagents, followed by acetylation with 5-20 equivalents of acetic anhydride/N-methylmorpholine for 30-60 min. At the time of charging, the amino acid and HOBt were dissolved in DMF, and DIC was added dropwise for activation. The coupling reaction time was generally 45 min, and the deprotection time was 20 min. After completion of peptide chain synthesis, the resin was washed with DCM and dried.

Cleavage was performed using $V_{TFA}$:$V_{TIS}$:$V_{H2O}$=90: 5: 5 for 1 h. After cleavage, the spent resin was removed by filtration. The filtrate was added to methyl tert-butyl ether which had been cooled at - 20°C and centrifuged. The supernatant was discarded, and the solid was washed more than twice with cold methyl tert-butyl ether. A small amount of sample was taken to analyze the molecular weight of the crude product by MS, and then the peptide was dissolved in 10-20 mL of acetonitrile-water and lyophilized. The crude peptide was dissolved in approximately 15% acetonitrile-water and loaded onto a C8 preparative HPLC column at a flow rate of 13 mL/min, using a 0.1% TFA/acetonitrile mobile phase and a gradient of 28%-48% acetonitrile for 30 min. Peptides with a purity greater than 85% (confirmed by MS and HPLC) were pooled and lyophilized to obtain the peptide powder (referred to as the naked peptide).

The naked peptide was dissolved in acetonitrile and PBS (pH 7.0) to obtain a solution containing about 1 mg per mL. A small amount of the solution was taken for analysis by HPLC to determine the retention time. Then, the naked peptide solution was added dropwise to a solution of VcMMAE (1.1 times the amount of naked peptide) dissolved in acetonitrile and PBS (pH 7.0), and the mixture was stirred for 30 min. After the reaction, a small amount of the solution was taken for analysis by HPLC to compare the retention time with that of the naked peptide. If the retention time shifted back and the MS results showed an error within $\pm 1.0$ of the theoretical molecular weight, the reaction was considered successful. The reaction mixture was diluted by one-fold with pure water and loaded onto a C8 preparative HPLC column at a flow rate of 13 mL/min, using a 0.1% TFA/acetonitrile mobile phase and a gradient of 40%-60% acetonitrile for 30 min. PDCs with a purity greater than 90% were pooled, lyophilized, and stored at -20°C.

**Example 2**

Synthesis of **PEG$_6$-Cys(MC-Val-Cit-PABC-MMAE)-PEG$_6$-Arg-Arg-Leu-Phe-[Cys-Tyr-D-Trp-Lys-Thr-Cys]-Thr-Trp-Arg-Asp-NH$_2$**

Peptide synthesis was based on Fmoc chemistry using a solid-phase synthesis method and 0.2 mmol/g Rink Amide-AM Resin and standard Fmoc-amino acid. The coupling agents used were DIC and HOBt, and deprotection was carried out with 20% piperidine in DMF. The resin modification was carried out using a 1.5-fold excess of Fmoc-Asp(OtBu)-OH and a 1.5-fold excess of coupling reagents.

Fmoc-Arg(Pbf)-OH, Fmoc-Trp(Boc)-OH, Fmoc-Thr(tBu)-OH, Fmoc-Cys(Trt)-OH, Fmoc-Thr(tBu)-OH, Fmoc-Lys(Boc)-OH, Fmoc-D-Trp(Boc)-OH, Fmoc-Tyr(tBu)-OH, and Fmoc-Cys(Trt)-OH were sequentially condensed using a 3-fold excess of Fmoc-amino acid and a 3-fold excess of coupling reagents. Solid phase cyclization was carried out with a 5-fold amount of iodine dissolved in DMF for 45 min, and after completion of the reaction, the resin was washed with $V_{DMF}$: $V_{ACN}$:$V_{H2O}$=90: 5: 5 and then washed three times with DMF.

Then, Fmoc-Phe-OH, Fmoc-Leu-OH, Fmoc-Arg(Pbf)-OH, Fmoc-Arg(Pbf)-OH, Fmoc-NH-PEG6-CH2CH2COOH, Fmoc-Cys(Trt)-OH, and Fmoc-NH-PEG$_6$-CH$_2$CH$_2$COOH were sequentially condensed using a 3-fold excess of Fmoc-amino acid and a 3-fold excess of coupling reagents. At the time of charging, the amino acid and HOBt were dissolved in DMF, and DIC was added dropwise for activation. The coupling reaction time was generally 45 min, and the deprotection time was 20 min. After completion of peptide chain synthesis, the resin was washed with DCM and dried.

Cleavage was performed using $V_{TFA}$: $V_{TIS}$:$V_{H2O}$=90: 5: 5 for 1 h. After cleavage, the spent resin was removed by filtration. The filtrate was added to methyl tert-butyl ether which had been cooled at - 20°C and centrifuged. The supernatant was discarded, and the solid was washed more than twice with cold methyl tert-butyl ether. A small amount of sample was taken to analyze the molecular weight of the crude product by MS, and then the peptide was dissolved in 10-20 mL of acetonitrile-water and lyophilized. The crude peptide was dissolved in approximately 15% acetonitrile-water and loaded onto a C8 preparative HPLC column at a flow rate of 13 mL/min, using a 0.1% TFA/acetonitrile mobile phase and a gradient of 20%-40% acetonitrile for 30 min. Peptides with a purity greater than 85%(confirmed by MS and HPLC) were pooled and lyophilized to obtain the peptide powder (referred to as the naked peptide).

The naked peptide was dissolved in acetonitrile and PBS (pH 7.0) to obtain a solution containing about 1 mg per mL. A small amount of the solution was taken for analysis by HPLC to determine the retention time. Then, the naked peptide solution was added dropwise to a solution of VcMMAE (1.1 times the amount of naked peptide) dissolved in acetonitrile and PBS (pH 7.0), and the mixture was stirred for 30 min. After the reaction, a small amount of the solution was taken for analysis by HPLC to compare the retention time with that of the naked peptide. If the retention time shifted back and the MS results showed an error within $\pm 1.0$ of the theoretical molecular weight, the reaction was considered successful. The reaction mixture was diluted by one-fold with pure water and loaded onto a C8 preparative HPLC column at a flow rate of 13 mL/min, using a 0.1% acetic acid/acetonitrile mobile phase and a gradient of 30%-50% acetonitrile for 30 min. The system was washed with 95% 20 mmol/L ammonium acetate and 5% acetonitrile for 20 min. PDCs with a purity greater than 90% were pooled, lyophilized, and stored at -20°C.

**Example 3**

Synthesis of **Ac-Cys(MC-Val-Cit-PABC-MMAE)-PEG$_6$-Arg-Arg-Leu-Phe-[Cys-Tyr-D-Trp-Lys-Thr-Cys]-Thr-Trp-Arg-Asp-NH$_2$**

Peptide synthesis was based on Fmoc chemistry using a solid-phase synthesis method and 0.2 mmol/g Rink Amide-AM Resin and standard Fmoc-amino acid. The coupling agents used were DIC and HOBt, and deprotection was carried out with 20% piperidine in DMF. The resin modification was carried out using a 1.5-fold excess of Fmoc-Asp(OtBu)-OH and a 1.5-fold excess of coupling reagents.

Fmoc-Arg(Pbf)-OH, Fmoc-Trp(Boc)-OH, Fmoc-Thr(tBu)-OH, Fmoc-Cys(Trt)-OH, Fmoc-Thr(tBu)-OH, Fmoc-Lys(Boc)-OH, Fmoc-D-Trp(Boc)-OH, Fmoc-Tyr(tBu)-OH, and Fmoc-Cys(Trt)-OH were sequentially condensed using a 3-fold excess of Fmoc-amino acid and a 3-fold excess of coupling reagents. Solid phase cyclization was carried out with a 5-fold amount of iodine dissolved in DMF for 45 min, and after completion of the reaction, the resin was washed with $V_{DMF}:V_{ACN}:V_{H2O}$=90: 5: 5 and then washed three times with DMF.

Then, Fmoc-Phe-OH, Fmoc-Leu-OH, Fmoc-Arg(Pbf)-OH, Fmoc-Arg(Pbf)-OH, Fmoc-NH-PEG$_6$-CH$_2$CH$_2$COOH, and Fmoc-Cys(Trt)-OH were sequentially condensed using a 3-fold excess of Fmoc-amino acid and a 3-fold excess of coupling reagents, followed by acetylation. At the time of charging, the amino acid and HOBt were dissolved in DMF, and DIC was added dropwise for activation. The coupling reaction time was generally 45 min, and the deprotection time was 20 min. After completion of peptide chain synthesis, the resin was washed with DCM and dried.

Cleavage was performed using $V_{TFA}:V_{TIS}:V_{H2O}$=90: 5: 5 for 1 h. After cleavage, the spent resin was removed by filtration. The filtrate was added to methyl tert-butyl ether which had been cooled at - 20°C and centrifuged. The supernatant was discarded, and the solid was washed more than twice with cold methyl tert-butyl ether. A small amount of sample was taken to analyze the molecular weight of the crude product by MS, and then the peptide was dissolved in 10-20 mL of acetonitrile-water and lyophilized. The crude peptide was dissolved in approximately 15% acetonitrile-water and loaded onto a C8 preparative HPLC column at a flow rate of 13 mL/min, using a 0.1% TFA/acetonitrile mobile phase and a gradient of 27%-47% acetonitrile for 30 min. Peptides with a purity greater than 85%(confirmed by MS and HPLC) were pooled and lyophilized to obtain the peptide powder (referred to as the naked peptide). The naked peptide was dissolved in acetonitrile and PBS (pH 7.0) to obtain a solution containing about 1 mg per mL. A small amount of the solution was taken for analysis by HPLC to determine the retention time. Then, the naked peptide solution was added dropwise to a solution of VcMMAE (1.1 times the amount of naked peptide) dissolved in acetonitrile and PBS (pH 7.0), and the mixture was stirred for 30 min. After the reaction, a small amount of the solution was taken for analysis by HPLC to compare the retention time with that of the naked peptide. If the retention time shifted back and the MS results showed an error within ±1.0 of the theoretical molecular weight, the reaction was considered successful. The reaction mixture was diluted by one-fold with pure water and loaded onto a C8 preparative HPLC column at a flow rate of 13 mL/min, using a 0.1% acetic acid/acetonitrile mobile phase and a gradient of 30%-50% acetonitrile for 30 min. The system was washed with 95% 20 mmol/L ammonium acetate and 5% acetonitrile for 20 min. PDCs with a purity greater than 90% were pooled, lyophilized, and stored at -20°C.

**Example 4**

**Synthesis of Ac-Cys(MC-betaglucuronide-MMAE)-AEEA-AEEA-AEEA-Arg-Arg-Leu-Phe-[Cys-Tyr-D-Trp-Lys-Thr-Cys]-Thr-Trp-Arg-Asp-NH$_2$**

Peptide synthesis was based on Fmoc chemistry using a solid-phase synthesis method and 0.2 mmol/g Rink Amide-AM Resin and standard Fmoc-amino acid. The coupling agents used were DIC and HOBt, and deprotection was carried out with 20% piperidine in DMF. The resin modification was carried out using a 1.5-fold excess of Fmoc-Asp(OtBu)-OH and a 1.5-fold excess of coupling reagents.

Fmoc-Arg(Pbf)-OH, Fmoc-Trp(Boc)-OH, Fmoc-Thr(tBu)-OH, Fmoc-Cys(Trt)-OH, Fmoc-Thr(tBu)-OH, Fmoc-Lys(Boc)-OH, Fmoc-D-Trp(Boc)-OH, Fmoc-Tyr(tBu)-OH, and Fmoc-Cys(Trt)-OH were sequentially condensed using a 3-fold excess of Fmoc-amino acid and a 3-fold excess of coupling reagents. Solid phase cyclization was carried out with a 5-fold amount of iodine dissolved in DMF for 45 min, and after completion of the reaction, the resin was washed with $V_{DMF}:V_{ACN}:V_{H2O}$=90: 5: 5 and then washed three times with DMF.

Then, Fmoc-Phe-OH, Fmoc-Leu-OH, Fmoc-Arg(Pbf)-OH, Fmoc-Arg(Pbf)-OH, AEEA, AEEA, AEEA, and Fmoc-Cys(Trt)-OH were sequentially condensed using a 3-fold excess of Fmoc-amino acid and a 3-fold excess of coupling reagents, followed by acetylation. At the time of charging, the amino acid and HOBt were dissolved in DMF, and DIC was added dropwise for activation. The coupling reaction time was generally 45 min, and the deprotection time was 20 min. After completion of peptide chain synthesis, the resin was washed with DCM and dried.

Cleavage was performed using $V_{TFA}:V_{TIS}:V_{H2O}$= 90:5:5 for 1 h. After cleavage, the spent resin was removed by filtration. The filtrate was added to methyl tert-butyl ether which had been cooled at - 20°C and centrifuged. The supernatant was discarded, and the solid was washed more than twice with cold methyl tert-butyl ether. A small amount of sample was taken to analyze the molecular weight of the crude product by MS, and then the peptide was dissolved in 10-20 mL of acetonitrile-water and lyophilized. The crude peptide was dissolved in approximately 15% acetonitrile-water and loaded onto a C8 preparative HPLC column at a flow rate of 13 mL/min, using a 0.1% TFA/acetonitrile mobile phase and a gradient of 25%-45% acetonitrile for 30 min. Peptides with a purity greater than 85%(confirmed by MS and HPLC) were pooled and lyophilized to obtain the peptide powder (referred to as the naked peptide).

The naked peptide was dissolved in acetonitrile and PBS (pH 7.0) to obtain a solution containing about 1 mg per mL. A small amount of the solution was taken for analysis by HPLC to determine the retention time. Then, the naked peptide solution was added dropwise to a solution of MC-betaglucuronide-MMAE-1 (1.1 times the amount of naked peptide) dissolved in acetonitrile and PBS (pH 7.0), and the mixture was stirred for 30 min. After the reaction, a small amount of the solution was taken for analysis by HPLC to compare the retention time with that of the naked peptide. If the retention time shifted back and the MS results showed an error within $\pm 1.0$ of the theoretical molecular weight, the reaction was considered successful. The reaction mixture was diluted by one-fold with pure water and loaded onto a C8 preparative HPLC column at a flow rate of 13 mL/min, using a 0.1% acetic acid/acetonitrile mobile phase and a gradient of 28%-48% acetonitrile for 30 min. The system was washed with 95% 20 mmol/L ammonium acetate and 5% acetonitrile for 20 min. PDCs with a purity greater than 90% were pooled, lyophilized, and stored at -20°C.

**Example 5**

**Synthesis of PEG$_6$-Cys(MC-Gly-Gly-Phe-Gly-NH-CH$_2$-Dxd)-PEG$_6$-Arg-Arg-Leu-Phe-[Cys-Tyr-D-Trp-Lys-Thr-Cys]-Thr-Trp-Arg-Asp-NH$_2$**

Peptide synthesis was based on Fmoc chemistry using a solid-phase synthesis method and 0.2 mmol/g Rink Amide-AM Resin and standard Fmoc-amino acid. The coupling agents used were DIC and HOBt, and deprotection was carried out with 20% piperidine in DMF. The resin modification was carried out using a 1.5-fold excess of Fmoc-Asp(OtBu)-OH and a 1.5-fold excess of coupling reagents.

Fmoc-Arg(Pbf)-OH, Fmoc-Trp(Boc)-OH, Fmoc-Thr(tBu)-OH, Fmoc-Cys(Trt)-OH, Fmoc-Thr(tBu)-OH, Fmoc-Lys(Boc)-OH, Fmoc-D-Trp(Boc)-OH, Fmoc-Tyr(tBu)-OH, and Fmoc-Cys(Trt)-OH were sequentially condensed using a 3-fold excess of Fmoc-amino acid and a 3-fold excess of coupling reagents. Solid phase cyclization was carried out with a 5-fold amount of iodine dissolved in DMF for 45 min, and after completion of the reaction, the resin was washed with $V_{DMF}:V_{ACN}:V_{H2O}= 90:5:5$ and then washed three times with DMF.

Then, Fmoc-Phe-OH, Fmoc-Leu-OH, Fmoc-Arg(Pbf)-OH, Fmoc-Arg(Pbf)-OH, Fmoc-NH-PEG6-CH2CH2COOH, Fmoc-Cys(Trt)-OH, and Fmoc-NH-PEG6-CH2CH2COOH were sequentially condensed using a 3-fold excess of Fmoc-amino acid and a 3-fold excess of coupling reagents. At the time of charging, the amino acid and HOBt were dissolved in DMF, and DIC was added dropwise for activation. The coupling reaction time was generally 45 min, and the deprotection time was 20 min. After completion of peptide chain synthesis, the resin was washed with DCM and dried.

Cleavage was performed using $V_{TFA}:V_{TIS}:V_{H2O}= 90:5:5$ for 1 h. After cleavage, the spent resin was removed by filtration. The filtrate was added to methyl tert-butyl ether which had been cooled at - 20°C and centrifuged. The supernatant was discarded, and the solid was washed more than twice with cold methyl tert-butyl ether. A small amount of sample was taken to analyze the molecular weight of the crude product by MS, and then the peptide was dissolved in 10-20 mL of acetonitrile-water and lyophilized. The crude peptide was dissolved in approximately 15% acetonitrile-water and loaded onto a C8 preparative HPLC column at a flow rate of 13 mL/min, using a 0.1% TFA/acetonitrile mobile phase and a gradient of 20%-40% acetonitrile for 30 min. Peptides with a purity greater than 85%(confirmed by MS and HPLC) were pooled and lyophilized to obtain the peptide powder (referred to as the naked peptide).

The naked peptide was dissolved in acetonitrile and PBS (pH 7.0) to obtain a solution containing about 1 mg per mL. A small amount of the solution was taken for analysis by HPLC to determine the retention time. Then, the naked peptide solution was added dropwise to a solution of MC-Gly-Gly-Phe-Gly-NH-CH$_2$-Dxd (1.1 times the amount of naked peptide) dissolved in acetonitrile and PBS (pH 7.0), and the mixture was stirred for 30 min. After the reaction, a small amount of the solution was taken for analysis by HPLC to compare the retention time with that of the naked peptide. If the retention time shifted back and the MS results showed an error within $\pm 1.0$ of the theoretical molecular weight, the reaction was considered successful. The reaction mixture was diluted by one-fold with pure water and loaded onto a C8 preparative HPLC column at a flow rate of 13 mL/min, using a 0.1% acetic acid/acetonitrile mobile phase and a gradient of 21%-41% acetonitrile for 30 min. The system was washed with 95% 20 mmol/L ammonium acetate and 5% acetonitrile for 20 min. PDCs with a purity greater than 90% were pooled, lyophilized, and stored at -20°C.

**Example 6**

**Synthesis of Ac-Cys(MC-Gly-Gly-Phe-Gly-NH-CH$_2$-Dxd)-PEG$_6$-Arg-Arg-Leu-Phe-[Cys-Tyr-D-Trp-Lys-Thr-Cys]-Thr-Trp-Arg-Asp-NH$_2$**

Peptide synthesis was based on Fmoc chemistry using a solid-phase synthesis method and 0.2 mmol/g Rink Amide-AM Resin and standard Fmoc-amino acid. The coupling agents used were DIC and HOBt, and deprotection was carried out with 20% piperidine in DMF. The resin modification was carried out using a 1.5-fold excess of Fmoc-Asp(OtBu)-OH and a 1.5-fold excess of coupling reagents.

Fmoc-Arg(Pbf)-OH, Fmoc-Trp(Boc)-OH, Fmoc-Thr(tBu)-OH, Fmoc-Cys(Trt)-OH, Fmoc-Thr(tBu)-OH, Fmoc-Lys(Boc)-OH, Fmoc-D-Trp(Boc)-OH, Fmoc-Tyr(tBu)-OH, and Fmoc-Cys(Trt)-OH were sequentially condensed using a 3-fold excess of Fmoc-amino acid and a 3-fold excess of coupling reagents. Solid phase cyclization was carried out with a 5-fold amount of iodine dissolved in DMF for 45 min, and after completion of the reaction,the resin was washed with $V_{DMF}:V_{ACN}:V_{H2O}= 90:5:5$ and then washed three times with DMF.

Then, Fmoc-Phe-OH, Fmoc-Leu-OH, Fmoc-Arg(Pbf)-OH, Fmoc-Arg(Pbf)-OH, Fmoc-NH-PEG$_6$-CH$_2$CH$_2$COOH, and Fmoc-Cys(Trt)-OH were sequentially condensed using a 3-fold excess of Fmoc-amino acid and a 3-fold excess of coupling reagents, followed by acetylation. At the time of charging, the amino acid and HOBt were dissolved in DMF, and DIC was added dropwise for activation. The coupling reaction time was generally 45 min, and the deprotection time was 20 min. After completion of peptide chain synthesis, the resin was washed with DCM and dried.

Cleavage was performed using $V_{TFA}:V_{TIS}:V_{H2O}= 90:5:5$ for 1 h. After cleavage, the spent resin was removed by filtration. The filtrate was added to methyl tert-butyl ether which had been cooled at - 20°C and centrifuged. The supernatant was discarded, and the solid was washed more than twice with cold methyl tert-butyl ether. A small amount of sample was taken to analyze the molecular weight of the crude product by MS, and then the peptide was dissolved in 10-20 mL of acetonitrile-water and lyophilized. The crude peptide was dissolved in approximately 15% acetonitrile-water and loaded onto a C8 preparative HPLC column at a flow rate of 13 mL/min, using a 0.1% TFA/acetonitrile mobile phase and a gradient of 27%-47% acetonitrile for 30 min. Peptides with a purity greater than 85%(confirmed by MS and HPLC) were pooled and lyophilized to obtain the peptide powder (referred to as the naked peptide).

The naked peptide was dissolved in acetonitrile and PBS (pH 7.0) to obtain a solution containing about 1 mg per mL. A small amount of the solution was taken for analysis by HPLC to determine the retention time. Then, the naked peptide solution was added dropwise to a solution of MC-Gly-Gly-Phe-Gly-NH-CH$_2$-Dxd (1.1 times the amount of naked peptide) dissolved in acetonitrile and PBS (pH 7.0), and the mixture was stirred for 30 min. After the reaction, a small amount of the solution was taken for analysis by HPLC to compare the retention time with that of the naked peptide. If the retention time shifted back and the MS results showed an error within $\pm 1.0$ of the theoretical molecular weight, the reaction was considered successful. The reaction mixture was diluted by one-fold with pure water and loaded onto a C8 preparative HPLC column at a flow rate of 13 mL/min, using a 0.1% acetic acid/acetonitrile mobile phase and a gradient of 24%-44% acetonitrile for 30 min. The system was washed with 95% 20 mmol/L ammonium acetate and 5% acetonitrile for 20 min. PDCs with a purity greater than 90% were pooled, lyophilized, and stored at -20°C.

**Example 7**

**Synthesis of Ac-Cys(MC-Gly-Gly-Phe-Gly-NH-CH$_2$-Dxd)-PEG6-Lys(Ac-Cys(MC-Gly-Gly-Phe-Gly-NH-CH$_2$-Dxd)-PEG$_6$)-Lys(Ac-Cys(MC-Gly-Gly-Phe-Gly-NH-CH$_2$-Dxd)-PEG6)-PEG$_6$-Arg-Arg-Leu-Phe-[Cys-Tyr-D-Trp-Lys-Thr-Cys]-Thr-Trp-Arg-Asp-NH$_2$**

Peptide synthesis was based on Fmoc chemistry using a solid-phase synthesis method and 0.2 mmol/g Rink Amide-AM Resin and standard Fmoc-amino acid. The coupling agents used were DIC and HOBt, and deprotection was carried out with 20% piperidine in DMF. The resin modification was carried out using a 1.5-fold excess of Fmoc-Asp (OtBu)-OH and a 1.5-fold excess of coupling reagents.

Fmoc-Arg(Pbf)-OH, Fmoc-Trp(Boc)-OH, Fmoc-Thr(tBu)-OH, Fmoc-Cys(Trt)-OH, Fmoc-Thr(tBu)-OH, Fmoc-Lys(Boc)-OH, Fmoc-D-Trp(Boc)-OH, Fmoc-Tyr(tBu)-OH, and Fmoc-Cys(Trt)-OH were sequentially condensed using a 3-fold excess of Fmoc-amino acid and a 3-fold excess of coupling reagents. Solid phase cyclization was carried out with a 5-fold amount of iodine dissolved in DMF for 45 min, and after completion of the reaction, the resin was washed with $V_{DMF}:V_{ACN}:V_{H2O}=90: 5: 5$ and then washed three times with DMF.

Then, Fmoc-Phe-OH, Fmoc-Leu-OH, Fmoc-Arg(Pbf)-OH, Fmoc-Arg(Pbf)-OH, Fmoc-NH-PEG$_6$-CH$_2$CH$_2$COOH, Fmoc-Lys(Dde)-OH, Fmoc-Lys(Dde)-OH, Fmoc-NH-PEG$_6$-CH$_2$CH$_2$COOH, and Fmoc-Cys(Trt)-OH were sequentially condensed using a 3-fold excess of Fmoc-amino acid and a 3-fold excess of coupling reagents, followed by acetylation. At the time of charging, the amino acid and HOBt were dissolved in DMF, and DIC was added dropwise for activation. The coupling reaction time was generally 45 min, and the deprotection time was 20 min.

The Dde protecting group was removed using 15% hydrazine hydrate/DMF. Then, Fmoc-NH-PEG$_6$-CH$_2$CH$_2$COOH and Fmoc-Cys(Trt)-OH were sequentially condensed using a 6-fold excess of starting material and a 6-fold excess of coupling reagents, followed by acetylation. After completion of peptide chain synthesis, the resin was washed with DCM and dried.

Cleavage was performed using $V_{TFA}:V_{TIS}:V_{H2O}=90: 5: 5$ for 1 h. After cleavage, the spent resin was removed by filtration. The filtrate was added to methyl tert-butyl ether which had been cooled at - 20°C and centrifuged. The supernatant was discarded, and the solid was washed more than twice with cold methyl tert-butyl ether. A small amount of sample was taken to analyze the molecular weight of the crude product by MS, and then the peptide was dissolved in 10-20 mL of acetonitrile-water and lyophilized. The crude peptide was dissolved in approximately 15% acetonitrile-water and loaded onto a C8 preparative HPLC column at a flow rate of 13 mL/min, using a 0.1% TFA/acetonitrile mobile phase and a gradient of 20%-40% acetonitrile for 30 min. Peptides with a purity greater than 85%(confirmed by MS and HPLC) were pooled and lyophilized to obtain the peptide powder (referred to as the naked peptide).

The naked peptide was dissolved in acetonitrile and PBS (pH 7.0) to obtain a solution containing about 1 mg per mL. A small amount of the solution was taken for analysis by HPLC to determine the retention time. Then, the naked peptide solution was added dropwise to a solution of MC-Gly-Gly-Phe-Gly-NH-CH$_2$-Dxd (3.3 times the amount of naked peptide) dissolved in acetonitrile and PBS (pH 7.0), and the mixture was stirred for 30 min. After the reaction, a small amount of the solution was taken for analysis by HPLC to compare the retention time with that of the naked peptide. If the retention time shifted back and the MS results showed an error within ±1.0 of the theoretical molecular weight, the reaction was considered successful. The reaction mixture was diluted by one-fold with pure water and loaded onto a C8 preparative HPLC column at a flow rate of 13 mL/min, using a 0.1% acetic acid/acetonitrile mobile phase and a gradient of 30%-50% acetonitrile for 30 min. The system was washed with 95% 20 mmol/L ammonium acetate and 5% acetonitrile for 20 min. PDCs with a purity greater than 90% were pooled, lyophilized, and stored at -20°C.

Example 8

Synthesis of **Ac-Cys(MC-Val-Cit-PABC-MMAE)-AEEA-AEEA-AEEA-Arg-Arg-Leu-Phe-[Cys-Phe-D-Trp-Lys-Thr-Cys]-Thr-Trp-Arg-Asp-NH$_2$**

Peptide synthesis was based on Fmoc chemistry using a solid-phase synthesis method and 0.2 mmol/g Rink Amide-AM Resin and standard Fmoc-amino acid. The coupling agents used were DIC and HOBt, and deprotection was carried out with 20% piperidine in DMF. The resin modification was carried out using a 1.5-fold excess of Fmoc-Asp(OtBu)-OH and a 1.5-fold excess of coupling reagents.

Fmoc-Arg(Pbf)-OH, Fmoc-Trp(Boc)-OH, Fmoc-Thr(tBu)-OH, Fmoc-Cys(Trt)-OH, Fmoc-Thr(tBu)-OH, Fmoc-Lys(Boc)-OH, Fmoc-D-Trp(Boc)-OH, Fmoc-Phe-OH, and Fmoc-Cys(Trt)-OH were sequentially condensed using a 3-fold excess of Fmoc-amino acid and a 3-fold excess of coupling reagents. Solid phase cyclization was carried out with a 5-fold amount of iodine dissolved in DMF for 45 min, and after completion of the reaction, the resin was washed with $V_{DMF}:V_{ACN}:V_{H2O}$=90: 5: 5 and then washed three times with DMF.

Then, Fmoc-Phe-OH, Fmoc-Leu-OH, Fmoc-Arg(Pbf)-OH, Fmoc-Arg(Pbf)-OH, AEEA, AEEA, AEEA, and Fmoc-Cys(Trt)-OH were sequentially condensed using a 3-fold excess of Fmoc-amino acid and a 3-fold excess of coupling reagents, followed by acetylation. At the time of charging, the amino acid and HOBt were dissolved in DMF, and DIC was added dropwise for activation. The coupling reaction time was generally 45 min, and the deprotection time was 20 min. After completion of peptide chain synthesis, the resin was washed with DCM and dried.

Cleavage was performed using $V_{TFA}:V_{TIS}:V_{H2O}$=90: 5: 5 for 1 h. After cleavage, the spent resin was removed by filtration. The filtrate was added to methyl tert-butyl ether which had been cooled at - 20°C and centrifuged. The supernatant was discarded, and the solid was washed more than twice with cold methyl tert-butyl ether. A small amount of sample was taken to analyze the molecular weight of the crude product by MS, and then the peptide was dissolved in 10-20 mL of acetonitrile-water and lyophilized. The crude peptide was dissolved in approximately 15% acetonitrile-water and loaded onto a C8 preparative HPLC column at a flow rate of 13 mL/min, using a 0.1% TFA/acetonitrile mobile phase and a gradient of 27%-47% acetonitrile for 30 min. Peptides with a purity greater than 85%(confirmed by MS and HPLC) were pooled and lyophilized to obtain the peptide powder (referred to as the naked peptide).

The naked peptide was dissolved in acetonitrile and PBS (pH 7.0) to obtain a solution containing about 1 mg per mL. A small amount of the solution was taken for analysis by HPLC to determine the retention time. Then, the naked peptide solution was added dropwise to a solution of VcMMAE (1.1 times the amount of naked peptide) dissolved in acetonitrile and PBS (pH 7.0), and the mixture was stirred for 30 min. After the reaction, a small amount of the solution was taken for analysis by HPLC to compare the retention time with that of the naked peptide. If the retention time shifted back and the MS results showed an error within ±1.0 of the theoretical molecular weight, the reaction was considered successful. The reaction mixture was diluted by one-fold with pure water and loaded onto a C8 preparative HPLC column at a flow rate of 13 mL/min, using a 0.1% acetic acid/acetonitrile mobile phase and a gradient of 33%-53% acetonitrile for 30 min. The system was washed with 95% 20 mmol/L ammonium acetate and 5% acetonitrile for 20 min. PDCs with a purity greater than 90% were pooled, lyophilized, and stored at -20°C.

**Example 9**

**Synthesis of Cys(MC-Val-Cit-PABC-MMAE)-Glu-Glu-Glu-AEEA-Arg-Arg-Leu-Phe-[Cys-Tyr-D-Trp-Lys-Thr-Cys]-Thr-Trp-Arg-Asp-NH$_2$**

Peptide synthesis was based on Fmoc chemistry using a solid-phase synthesis method and 0.2 mmol/g Rink Amide-AM Resin and standard Fmoc-amino acid. The coupling agents used were DIC and HOBt, and deprotection was carried out with 20% piperidine in DMF. The resin modification was carried out using a 1.5-fold excess of Fmoc-Asp(OtBu)-OH and a 1.5-fold excess of coupling reagents.

Fmoc-Arg(Pbf)-OH, Fmoc-Trp(Boc)-OH, Fmoc-Thr(tBu)-OH, Fmoc-Cys(Trt)-OH, Fmoc-Thr(tBu)-OH, Fmoc-Lys(Boc)-OH, Fmoc-D-Trp(Boc)-OH, Fmoc-Tyr(tBu)-OH, and Fmoc-Cys(Trt)-OH were sequentially condensed using a 3-fold excess of Fmoc-amino acid and a 3-fold excess of coupling reagents. Solid phase cyclization was carried out with a 5-fold amount of iodine dissolved in DMF for 45 min, and after completion of the reaction, the resin was washed with $V_{DMF}:V_{ACN}:V_{H2O}$=90: 5: 5 and then washed three times with DMF.

Then,Fmoc-Phe-OH, Fmoc-Leu-OH, Fmoc-Arg(Pbf)-OH, Fmoc-Arg(Pbf)-OH, AEEA, Fmoc-Glu(OtBu)-OH, Fmoc-Glu(OtBu)-OH, Fmoc-Glu(OtBu)-OH, and Fmoc-Cys(Trt)-OH were sequentially condensed using a 3-fold excess of Fmoc-amino acid and a 3-fold excess of coupling reagents. At the time of charging, the amino acid and HOBt were dissolved in DMF, and DIC was added dropwise for activation. The coupling reaction time was generally 45 min, and the deprotection time was 20 min. After completion of peptide chain synthesis, the resin was washed with DCM and dried.

Cleavage was performed using $V_{TFA}:V_{TIS}:V_{H2O}$=90: 5: 5 for 1 h. After cleavage, the spent resin was removed by filtration. The filtrate was added to methyl tert-butyl ether which had been cooled at - 20°C and centrifuged. The supernatant was discarded, and the solid was washed more than twice with cold methyl tert-butyl ether. A small amount of sample was taken to analyze the molecular weight of the crude product by MS, and then the peptide was dissolved in 10-20 mL of acetonitrile-water and lyophilized. The crude peptide was dissolved in approximately 15% acetonitrile-water and loaded onto a C8 preparative HPLC column at a flow rate of 13 mL/min, using a 0.1% TFA/acetonitrile mobile phase and a gradient of 28%-48% acetonitrile for 30 min. Peptides with a purity greater than 85%(confirmed by MS and HPLC) were pooled and lyophilized to obtain the peptide powder (referred to as the naked peptide).

The naked peptide was dissolved in acetonitrile and PBS (pH 7.0) to obtain a solution containing about 1 mg per mL. A small amount of the solution was taken for analysis by HPLC to determine the retention time. Then, the naked peptide solution was added dropwise to a solution of VcMMAE (1.1 times the amount of naked peptide) dissolved in acetonitrile and PBS (pH 7.0), and the mixture was stirred for 30 min. After the reaction, a small amount of the solution was taken for analysis by HPLC to compare the retention time with that of the naked peptide. If the retention time shifted back and the MS results showed an error within $\pm 1.0$ of the theoretical molecular weight, the reaction was considered successful. The reaction mixture was diluted by one-fold with pure water and loaded onto a C8 preparative HPLC column at a flow rate of 13 mL/min, using a 0.1% TFA/acetonitrile mobile phase and a gradient of 41%-61% acetonitrile for 30 min. PDCs with a purity greater than 90% were pooled, lyophilized, and stored at -20°C.

**Example 10**

**Synthesis of Ac-Cys(MC-Val-Cit-PABC-MMAE)-AEEA-Arg-Arg-Leu-Phe-[Cys-Tyr-D-Trp-Lys-Thr-Cys]-Thr-Trp-Arg-Asp-NH$_2$**

Peptide synthesis was based on Fmoc chemistry using a solid-phase synthesis method and 0.2 mmol/g Rink Amide-AM Resin and standard Fmoc-amino acid. The coupling agents used were DIC and HOBt, and deprotection was carried out with 20% piperidine in DMF. The resin modification was carried out using a 1.5-fold excess of Fmoc-Asp(OtBu)-OH and a 1.5-fold excess of coupling reagents.

Fmoc-Arg(Pbf)-OH, Fmoc-Trp(Boc)-OH, Fmoc-Thr(tBu)-OH, Fmoc-Cys(Trt)-OH, Fmoc-Thr(tBu)-OH, Fmoc-Lys(Boc)-OH, Fmoc-D-Trp(Boc)-OH, Fmoc-Tyr(tBu)-OH, and Fmoc-Cys(Trt)-OH were sequentially condensed using a 3-fold excess of Fmoc-amino acid and a 3-fold excess of coupling reagents. Solid phase cyclization was carried out with a 5-fold amount of iodine dissolved in DMF for 45 min, and after completion of the reaction, the resin was washed with $V_{DMF}:V_{ACN}:V_{H2O}$=90: 5: 5 and then washed three times with DMF.

Then,Fmoc-Phe-OH, Fmoc-Leu-OH, Fmoc-Arg(Pbf)-OH, Fmoc-Arg(Pbf)-OH, AEEA, and Fmoc-Cys(Trt)-OH were sequentially condensed using a 3-fold excess of Fmoc-amino acid and a 3-fold excess of coupling reagents, followed by acetylation with 5-20 equivalents of acetic anhydride/N-methylmorpholine for 30-60 min. At the time of charging, the amino acid and HOBt were dissolved in DMF, and DIC was added dropwise for activation. The coupling reaction time was generally 45 min, and the deprotection time was 20 min. After completion of peptide chain synthesis, the resin was washed with DCM and dried.

Cleavage was performed using $V_{TFA}:V_{TIS}:V_{H2O}$=90: 5: 5 for 1 h. After cleavage, the spent resin was removed by filtration. The filtrate was added to methyl tert-butyl ether which had been cooled at - 20°C and centrifuged. The supernatant was discarded, and the solid was washed more than twice with cold methyl tert-butyl ether. A small amount of sample was taken to analyze the molecular weight of the crude product by MS, and then the peptide was dissolved in 10-20 mL of acetonitrile-water and lyophilized. The crude peptide was dissolved in approximately 15% acetonitrile-water and loaded onto a C8 preparative HPLC column at a flow rate of 13 mL/min, using a 0.1% TFA/acetonitrile mobile phase and a gradient of 28%-48% acetonitrile for 30 min. Peptides with a purity greater than 85%(confirmed by MS and HPLC) were pooled and lyophilized to obtain the peptide powder (referred to as the naked peptide).

The naked peptide was dissolved in acetonitrile and PBS (pH 7.0) to obtain a solution containing about 1 mg per mL. A small amount of the solution was taken for analysis by HPLC to determine the retention time. Then, the naked peptide solution was added dropwise to a solution of VcMMAE (1.1 times the amount of naked peptide) dissolved in acetonitrile and PBS (pH 7.0), and the mixture was stirred for 30 min. After the reaction, a small amount of the solution was taken for analysis by HPLC to compare the retention time with that of the naked peptide. If the retention time shifted back and the MS results showed an error within ±1.0 of the theoretical molecular weight, the reaction was considered successful. The reaction mixture was diluted by one-fold with pure water and loaded onto a C8 preparative HPLC column at a flow rate of 13 mL/min, using a 0.1% TFA/acetonitrile mobile phase and a gradient of 40%-60% acetonitrile for 30 min. PDCs with a purity greater than 90% were pooled, lyophilized, and stored at -20°C.

The sources or meanings of the compounds used in the above examples are as follows:

Table 1:Sources of Compounds Used in Examples 1-10

| Abbreviation | Full Name | Supplier |
|---|---|---|
| | Rink Amide-AM resin | Tianjin Nankai Hecheng Science & Technology Co., Ltd. |
| | Fmoc-Asp(OtBu)-OH | GL Biochem (Shanghai) Ltd. |
| | Fmoc-Arg(Pbf)-OH | |
| | Fmoc-Thr(tBu)-OH | |
| | Fmoc-Lys(Boc)-OH | |
| | Fmoc-Leu-OH | |
| | Fmoc-Glu(OtBu)-OH | |
| | Fmoc-Trp(Boc)-OH | Shanghai Plus Bio-Sci & Tech Co., Ltd. |
| | Fmoc-Cys(Trt)-OH | |
| | Fmoc-D-Trp(Boc)-OH | Chengdu Tachem Co., Ltd. |
| | Fmoc-Tyr(tBu)-OH | |
| | Fmoc-Phe-OH | |
| | Fmoc-NH-PEG6-CH2-CH2-COOH | Chengdu Pukang Biotechnology Technology Co., Ltd. |
| AEEA | [2-[2-(Fmoc-amino) ethoxy] ethoxy] acetic acid | Bide Pharmatech Ltd. |
| VcMMAE | MC-Val-Cit-PAB-MMAE | Shanghai Haoyuan Chemexpress Co., Ltd. |
| | MC-betaglucuronide-MMAE-1 | |
| | MC-Gly-Gly-Phe-Gly-NH-CH2-Dxd | |
| DMF | N, N-Dimethylformamide | Liaocheng Luxi Methylamine Chemical Co., Ltd. |
| DCM | Dichloromethane | Shandong Jinling Chemical Co., Ltd. |
| DIC | N,N'-Diisopropylcarbodiimide | Suzhon Highfine Biotech Co., Ltd. |
| HOBt | 1-Hydroxybenzotriazole | |
| PIP | Piperidine | Shandong Kunda Biotechnology Co., Ltd. |
| DIEA | N.N-Diisopropylethylamine | Hubei Nodina Biotechnology Co., Ltd. |
| TFA | Trifluoroacetic acid | Anaqua Chemicals Supply |
| TIS | Triisopropylsilane | Heowns Aopude (Tianjin) Technology Co., Ltd. |
| MTBE | Methyl tert-butyl ether | Jinan Aotai Chemical Co., Ltd. |
| ACN | Acetonitrile | Concord Technology (Tianjin) Co., Ltd. |
| | Acetic anhydride | Sinopharm Chemical Reagent Co., Ltd. |
| | Ammonium acetate | |
| GAA | Glacial acetic acid | |

**Effect Example 1: Target Affinity Test of PDC**

**SSTR2 cAMP Test:**

1) Compound preparation: The test compound was diluted by 4-fold with DMSO to obtain 10 different concentration points. Approximately 100 nL of the diluted compound was then transferred to a cell reaction plate and centrifuged for later use.
2) Cell preparation:The cultured SSTR2/CHO cells were collected into a sterile centrifuge tube, and the supernatant was discarded by centrifugation.
3) The cells were resuspended in assay buffer, counted using a Vi-cell counter, and diluted with assay buffer.
4) Approximately 100 nL of 100 $\mu$M Forskolin was transferred into the cell reaction plate using an Echo and centrifuged. Then, 10 $\mu$L of assay buffer containing the cells was added and incubated.
5) Approximately 10 $\mu$L of cAMP detection reagent solution was added to the corresponding wells of the cell reaction plate, and incubated at room temperature in the dark.
6) The plate was read using a microplate reader. The final value was determined as the ratio of OD at 665 nm to that at 615 nm.
7) The cAMP value was calculated for each well: Activity (%)=(signal value of sample well - mean signal value of low-signal control wells)/ (mean signal value of high-signal control wells - mean signal value of low-signal control wells). Fitting was performed using the "Nonlinear regression (curve fit)-log(agonist) vs. response -- Variable slope" model in GraphPad Prism 5.0, with the log-transformed compound concentration on the x-axis and cAMP activation rate on the y-axis. The $EC_{50}$ value of the sample was calculated accordingly.

Table 2: SSTR2 cAMP Test Results

| S/N | Reagent Used | EC50 (nM) | Max Dose (nM) | Activity (%) @Max Dose |
|---|---|---|---|---|
| 1 | Example 1 | 662.9 | 50000 | 93.9 |
| 2 | Example 2 | 476.8 | 50000 | 95.4 |
| 3 | Example 3 | 559.5 | 50000 | 95.4 |
| 4 | Example 4 | 720.2 | 50000 | 95.2 |
| 5 | Example 5 | 155.4 | 50000 | 83.5 |
| 6 | Example 6 | 147.7 | 50000 | 67.8 |
| 7 | Example 7 | 23360 | 50000 | 54.4 |
| 8 | Example 8 | 404.5 | 50000 | 98.4 |
| 9 | Cortistatin14 | 1.381 | 1000 | 99.7 |
| 10 | SEQ ID No. 1 | 217.4 | 100000 | 98.0 |
| 11 | SEQ ID No. 2 | 35.45 | 150000 | 99.1 |
| It can be seen that the peptide sequence of the present application exhibits good affinity for SSTR2. | | | | |

**Effect Example 2:Cell Killing Assay of PDC**

**Cell Killing in NCI-H69 by CGT:**

a) Cell line: NCI-H69

b) Procedure

(1) The suspended NCI-H69 cells were centrifuged, resuspended in fresh growth medium, and counted using a cell counter;

(2) The cells were diluted to 3.125 w/mL with growth medium;

(3) The cell suspension was inoculated into a 96-well plate at 95 $\mu$L/well;

(4) The PDC of the present application was diluted with the growth medium containing DMSO. Approximately 5 $\mu$L of the PDC was added to the corresponding wells of the plate, and compared with DMSO (control, without the PDC of the present application), PTX, MMAE, and Dxd wells. The final concentration of DMSO in each well was 0.2%;

(5) The cells were incubated in a 5% $CO_2$ incubator at 37°C for 96 h;

(6) The cell culture plate was equilibrated to room temperature, and 50 $\mu$L of CellTiter-Glo® Reagent was added to each well;

(7) The plate was placed on a shaker for 2 min to ensure complete cell lysis;

(8) After mixing, the plate was placed at room temperature to react for 10 min. The fluorescence value (in RLU) was read when the signal became stable.

c) Data analysis

(1) The inhibition (Inh %) relative to the DMSO control was calculated as follows: Inhibition (Inh%)= 100 -(RLU of compound - RLU of DMSO control)/ (RLU of DMSO control - RLU of blank)*100%

(2) The data were analyzed using Graphpad 5.0 software. A 4-parameter equation was fitted to generate a concentration-response curve to obtain the $IC_{50}$.

Table 3: Test Results of Cell Killing in NCI-H69

| Test Compound | Top (Inhibition at Highest Point) | Bottom (Inhibition at Lowest Point) | Hillslope (Curve Gradient/Slope) | $IC_{50}$(nM) |
|---|---|---|---|---|
| PTX | 78.37 | 0.47 | 2.18 | 3.74 |
| MMAE | 85.99 | 0.31 | 0.97 | 0.57 |
| Dxd | 98.19 | = 0.0 | 0.50 | 7.53 |
| Example 1 | 79.37 | 2.17 | 1.53 | 147.20 |
| Example 4 | 75.08 | 3.88 | 1.15 | 7.54 |
| Example 5 | 75.87 | 8.37 | 0.72 | 316.30 |
| Example 6 | 78.21 | 10.30 | 0.60 | 275.70 |
| Example 7 | 68.39 | 0.65 | 0.90 | 122.20 |

**Cell Killing in HL-60 by CCK-8:**

a) Cell line: HL-60

b) Procedure

(1) Normally cultured HL-60 cells in the logarithmic growth phase were centrifuged at 800 rpm for 5 min to discard the supernatant. Approximately 3 mL of normal medium was added to resuspend the cell pellet by gently pipetting up and down. The cells were then counted using a cell counter and inoculated onto a 96-well plate.

(2) Sample solutions with different concentration gradients were prepared. Approximately 10 $\mu$L of each solution was added to the 96-well plate, with 3 to 6 replicate wells for each concentration, and incubated in a 5% $CO_2$ incubator at 37°C for 48 h.

(3) CCK-8 was equilibrated to room temperature before use, and 10 $\mu$L of CCK-8 solution was added to each well.

(4) The plate was incubated in the incubator for 0.5-4 h in the dark, with the incubation time depending on experimental conditions such as cell type and density.

(5) The absorbance was measured at 450 nm using a microplate reader.

c) Data analysis

$$\text{Cell viability} = [(A_s - A_b)/(A_c - A_b)] \times 100\%$$

$$\text{Inhibition}\% = [(A_c - A_s)/(A_c - A_b)] \times 100\%$$

$A_s$: absorbance of sample well (containing cells + medium + CCK-8 solution + drug solution);
$A_c$: absorbance of control well (containing cells + medium + CCK-8 solution);
$A_b$: absorbance of blank well (containing medium + CCK-8 solution);
The results were processed and analyzed using Excel and Graphpad Prism to obtain the $IC_{50}$.

Table 4: Test Results of Cell Killing in HL-60 for 48 h

| Compound | Top (Inhibition at Highest Point) | Bottom (Inhibition at Lowest Point) | $IC_{50}$(nM) |
|---|---|---|---|
| Drug 1 | 86.42 | 1.71 | 203.8 |
| Drug 10 | 90.08 | -2.25 | 186.6 |

## Effect Example 3: *In Vivo* Efficacy Test of CDX

### EX3.1:*In Vivo* Efficacy of Test Article on NCI-H69 Cells in Balb/c Nude Mouse Model of Subcutaneous Tumors

Laboratory animals:

Species: Mice
Strain: Balb/c nude mice
Age: 6-8 weeks
Sex: Female
Animal source:Shanghai Jihui Laboratory Animal Care Co., Ltd.

**Procedure**

a) Cell culture

NCI-H69 cells were cultured in RPMI1640 medium containing 20% fetal bovine serum, 100 U/mL penicillin, and 100 $\mu$g/mL streptomycin in a 5% $CO_2$ incubator at 37°C, with 2-3 passages per week. The cells were collected after they grew to the logarithmic growth phase, washed twice with serum-free medium, and resuspended in serum-free medium. The cells were then collected, counted, and inoculated.

b) Animal grouping and administration

Each mouse was subcutaneously injected with 0.1 mL of NCI-H69 tumor cell suspension containing 50% Matrigel at right axilla ($5.00 \times 10^6$/animal, with the day of administration designated as Day 0). When the tumor volume reached about 100 mm$^3$, mice were selected and randomly divided into groups (vehicle control, positive control, and Example 4 treatment groups, with 6 animals per group)using Excel based on their tumor size and body weight. Each mouse received an intravenous injection of 5% DMSO + 95% normal saline, etoposide (10 mg/kg), or the drug of Example 4 (22.8 mg/kg), twice a week.

c) Tumor measurement

The mice were measured for body weight twice a week using an electronic balance. The tumor diameter was measured three times a week using a vernier caliper. The tumor volume was calculated as follows: $V = 0.5 \times a \times b^2$, where $a$ and $b$ were the length and width of the tumor, respectively.
Relative tumor proliferation rate:T/C (%)= $T_{RTV}/C_{RTV} \times 100\%$($T_{RTV}$: RTV of treatment group, $C_{RTV}$: RTV of vehicle control, RTV = $V_1/Vo$).
Tumor growth inhibition rate:

$$TGI\ (\%)=(1-T/C)\times100\%.$$

Table 5: Tumor Volume of Mice by Group ($mm^3$, Mean $\pm$ SEM)

| Day | Vehicle Control(5% DMSO + 95%Normal Saline) | Positive Control (Etoposide 10 mpk) | Example 4 (22.8mpk) |
|---|---|---|---|
| 18 | 149$\pm$13 | 148$\pm$12 | 149$\pm$11 |
| 21 | 290$\pm$39 | 197$\pm$12 | 187$\pm$24 |
| 25 | 478$\pm$88 | 283$\pm$31 | 150$\pm$44 |
| 28 | 701$\pm$192 | 346$\pm$55 | 149$\pm$47 |
| 32 | 945$\pm$317 | 423$\pm$101 | 114$\pm$36 |
| 35 | 1212$\pm$439 | 541$\pm$176 | 85$\pm$24 |
| 39 | 1050$\pm$338(n=5) | 740$\pm$243 | 40$\pm$12 |
| 42 | 1146$\pm$364(n=5) | 808$\pm$276 | 32$\pm$8 |
| 46 | 1022$\pm$66(n=4) | 1057$\pm$365 | 16$\pm$12 |

**Experimental results:**

The anti-tumor effect of the test article was evaluated in the BALB/c nude mouse model of subcutaneous tumors (NCI-H69 cells). By Day 32, both the positive control group (etoposide, 10 mg/kg) and Example 4 treatment group (22.8 mg/kg) showed significant tumor inhibition compared to the vehicle control group (p< 0.001). In the Example 4 treatment group, 2/6 of the mice achieved PR (partial response, tumor shrinkage by more than 50%), and 1/6 of the mice had no change (tumor shrinkage by less than 50% or increase by less than 25%). The TGI of this application was 87.90%.

**EX3.2:** *In Vivo* **Efficacy of Test Article on NCI-H69 Cells in Balb/c Nude Mouse Model of Subcutaneous Tumors**

**Laboratory animals:**

Balb/c nude mice, 6-8 weeks old, female, sourced from Shanghai Jihui Laboratory Animal Care Co., Ltd.

a) Cell culture

NCI-H69 cells were cultured in RPMI1640 medium containing 20% fetal bovine serum, 100 U/mL penicillin, and 100 $\mu$g/mL streptomycin in a 5% $CO_2$ incubator at 37°C, with 2-3 passages per week. The cells were collected after they grew to the logarithmic growth phase, washed twice with serum-free medium, and resuspended in serum-free medium. The cells were then collected, counted, and inoculated.

b) Animal grouping and administration

Each mouse was subcutaneously injected with 0.1 mL of NCI-H69 tumor cell suspension containing 50% Matrigel at right axilla ($5.00 \times 10^6$/animal, with the day of administration designated as Day 0). When the tumor volume reached about 100 $mm^3$, mice were selected and randomly divided into groups (vehicle control and Example 4 treatment groups, with 6 animals per group) using Excel based on their tumor size and body weight. Each mouse received an intravenous injection of 5% DMSO + 95% normal saline or the drug of Example 4 (53.7 mg/kg), with 4 consecutive days of injection followed by 3 days of rest as a cycle.

c) Tumor measurement

The mice were measured for body weight twice a week using an electronic balance. The tumor diameter was measured three times a week using a vernier caliper. The tumor volume was calculated as follows: $V = 0.5 \times a \times b^2$, where $a$ and $b$ were the length and width of the tumor, respectively.

Relative tumor proliferation rate:

$$T/C\ (\%) = T_{RTV}/C_{RTV} \times 100\%(RTV = V_t/V_0).$$

Tumor growth inhibition rate:

$$TGI\ (\%) = (1-T/C) \times 100\%.$$

The *in vivo* efficacy results of the test article on NCI-H69 cells in the Balb/c nude mouse model of subcutaneous tumors are shown in Table 5 below.

Table 5: Tumor Volume of Mice by Group (mm$^3$, Mean)

| Day | Vehicle Control(5% DMSO + 95%Normal Saline) | Drug of Example 4 |
|---|---|---|
| 16 | 307 | 307 |
| 20 | 509 | 376 |
| 23 | 557 | 376 |
| 27 | 586 | 238 |
| 30 | 863 | 135 |
| 34 | 1121 | 68 |

**Experimental results:**

The anti-tumor effect of the test article was evaluated in the BALB/c nude mouse model of subcutaneous tumors (NCI-H69 cells). By Day 34, the present application showed significant tumor inhibition compared to the vehicle control group (p< 0.001), with a TGI of 93.9%.

**EX3.3: *In Vivo* Pharmacodynamics of the Test Article on Human Multiple Myeloma MM.1S Cells in the CB17 SCID Mouse Model of Subcutaneous Xenograft Tumors**

**a) Cell culture:**

Human multiple myeloma MM.1S cells were suspended and cultured *in vitro* in 1640 medium containing 10% fetal bovine serum, 100 U/mL penicillin, and 100 $\mu$g/mL streptomycin in a 5% $CO_2$ incubator at 37°C. The suspended cells were collected by centrifugation twice a week and adherent cells were digested with trypsin-EDTA to prepare a cell suspension for subculture. When the cell saturation was 80%-90% and the cell number reached the requirement, the cells were collected, counted, and inoculated.

**b) Animals:**

CB17 SCID mice, female, 6-8 weeks old, weighing 18-22 g, provided by Zhejiang Vital River Laboratory Animal Technology Co., Ltd.

**c) Modeling:**

Each mouse was subcutaneously injected with 100 $\mu$L of PBS containing $5 \times 10^6$ MM.1S cells + 100 $\mu$L of Matrigel (final volume: 200 $\mu$L) at the axilla of the right forelimb. At 20 days after injection, when the average tumor volume reached 109 mm$^3$, mice were grouped for dosing (vehicle control, Ixazomib Citrate, and Example 1 treatment groups, with 6 animals per group). The Ixazomib Citrate group was orally administered by gavage (4 mg/kg) twice a week; the vehicle control group (5%DMSO + 95% normal saline) and the Example 1 treatment group (1.75 mg/kg) were slowly intravenously injected for about 30 seconds each time, twice a week.

**d) Experimental indicators:**

The experimental indicators were used to investigate whether tumor growth was inhibited, delayed, or cured. The tumor diameter was measured twice a week using a vernier caliper. The tumor volume was calculated as follows: V = $0.5a \times b^2$, where *a* and *b* were the long and short diameters of the tumor, respectively.

The anti-tumor efficacy of the drug was evaluated based on TGI (%) or relative tumor proliferation rate T/C (%). TGI (%) was the tumor growth inhibition rate. TGI (%) was calculated as follows: TGI (%)=[(1 -(mean tumor volume at the end of dosing in a certain treatment group - mean tumor volume at the start of dosing in the treatment group))/ (mean tumor volume at the end of dosing in the vehicle control group - mean tumor volume at the start of dosing in the vehicle control group)]$\times$ 100%.

T/C (%) was calculated as follows:T/C (%)= $T_{RTV}/ C_{RTV} \times$ 100 %. Based on the results of tumor measurement, the relative tumor volume (RTV) was calculated as follows: RTV = $V_t/V_0$, where $V_0$ was the mean tumor volume measured at the time of grouping (i.e. d0), and $V_t$ was the mean tumor volume at a certain measurement. Data on the same day were taken for $T_{RTV}$ and $C_{RTV}$.

Tumor weights were measured at the end of the experiment and $T/C_{weight}$ was calculated, where $T_{weight}$ and $C_{weight}$ were tumor weights in the treatment and vehicle control groups, respectively.

**e) Data analysis:**

Statistical analysis, including the mean and standard error (SEM) of tumor volume at each time point in each group (see Table 5-1 in Section 5.2 for specific data), was performed to evaluate differences between groups based on data from the treatment group on Day 18 post-dose at the end of the experiment. A t-test was used for comparison between two groups, and one-way ANOVA was used for comparison among three or more groups. If there was a significant difference in F value, the Games-Howell method was used for testing; if there was no significant difference in F value, the Dunnet (2-sided) method was used for analysis. All data were analyzed using SPSS 17.0. *P* < 0.05 was considered to be significantly different.

**f) Tumor volume:**

The mean change in tumor volume ($mm^3$) of each group after treatment with the test article in the female CB17 SCID mouse model of subcutaneous xenograft tumors (MM.1S cells) is shown in the table below.

Table 6:Tumor Volume of Mice by Group ($mm^3$,Mean $\pm$ SEM)

| Day | Vehicle Control (5% DMSO + 95% Normal Saline, IV,Twice Weekly) | Ixazomib Citrate (4 mg/kg,Oral Gavage,Twice Weekly) | Drug of Example 1 (1.75 mg/kg, IV,Twice Weekly) |
|---|---|---|---|
| 0 | 109 $\pm$ 7 | 109 $\pm$ 6 | 109 $\pm$ 6 |
| 4 | 321 $\pm$ 20 | 245 $\pm$ 23 | 144 $\pm$ 14 |
| 7 | 829 $\pm$ 89 | 337 $\pm$ 25 | 124 $\pm$ 13 |
| 11 | 1,346 $\pm$ 127 | 452 $\pm$ 59 | 87 $\pm$ 11 |
| 14 | 1,851 $\pm$ 223 | 586 $\pm$ 100 | 50 $\pm$ 8 |
| 18 | 2,487 $\pm$ 320 | 781 $\pm$ 177 | 23 $\pm$ 5 |
| 21 | 2,383 $\pm$ 382 | 925 $\pm$ 226 | 12 $\pm$ 5 |
| 25 | 2,512 $\pm$ 52 | 1,139 $\pm$ 271 | 0 $\pm$ 0 |
| 28 | 3,387 $\pm$ 130 | 1,365 $\pm$ 331 | 0 $\pm$ 0 |

**Experimental results:**

The *in vivo* efficacy of the test article was evaluated in the mouse model of subcutaneous xenograft tumors (MM.1 S cells). At 18 days after the start of dosing, the tumor volume of mice in the vehicle control group reached 2,487 $mm^3$. Compared to the vehicle control group, both the Ixazomib Citrate and Example 1 treatment groups showed significant tumor inhibition, with a tumor volume of 781 $mm^3$(T/C = 38.38%, TGI = 71.76%, *p*= 0.040) and 23 $mm^3$(T/C = 0.94%, TGI = 103.59%, *p*= 0.010), respectively. The 6 animals in the Example 1 treatment group all achieved a complete response. No significant weight loss or morbidity was observed in any dose group throughout the experiment.

In summary, the test article of the present application was tolerated in animals in this experiment. The drug of Example 1 showed a significant anti-tumor effect on MM.1S cells in the mouse model of subcutaneous xenograft tumors at 1.75 mg/kg, with a complete response rate of 100%.

The specific embodiments of the present application have been described in detail above, but they are merely examples, and the present application is not limited to the specific embodiments described above. For those skilled in the art, any equivalent modifications and substitutions to the present application are also within the scope of the present application. Therefore, all equivalent variations and modifications made without departing from the spirit and scope of the present application are to be encompassed within the scope of the present application.

**Claims**

1. A peptide targeting SSTR2,comprising the following sequence:
   Arg-X1-Phe-[Cys-Xaa-Trp-Lys-Thr-Cys]-Thr-X2-Arg-X3-NH$_2$/OH;

   wherein a disulfide bond is formed between the two Cys residues within the square brackets "[]";
   Xaa is selected one or more from a group consisting of Tyr and Phe; X1 and X2 are selected from any amino acid residues; X3 is selected from any amino acid residues or is absent.

2. The peptide targeting SSTR2 according to claim 1, wherein X1 is Arg or Leu, X2 is Trp or Asp, and X3 is Asp; Trp is D-Trp.

3. The peptide targeting SSTR2 according to claim 1, wherein the sequence is:
   X0-Arg-X1-Phe-[Cys-Xaa-Trp-Lys-Thr-Cys]-Thr-X2-Arg-X3-NH$_2$/OH;
   wherein X0 is selected from amino acid residues capable of reacting with an amino group or from other carboxyl-containing groups, and X0 is more preferably Arg or Gly.

4. The peptide targeting SSTR2 according to claim 3, wherein the peptide targeting SSTR2 is selected from the following sequences:

   SEQ ID NO. 1:
   H-Arg-Arg-Leu-Phe-[Cys-Tyr-Trp-Lys-Thr-Cys]-Thr-Trp-Arg-Asp-NH$_2$
   SEQ ID NO. 2:
   H-Arg-Arg-Leu-Phe-[Cys-Phe-Trp-Lys-Thr-Cys]-Thr-Trp-Arg-Asp-NH$_2$
   SEQ ID NO. 3:
   H-Gly-Arg-Arg-Phe-[Cys-Tyr-Trp-Lys-Thr-Cys]-Thr-Asp-Arg-NH$_2$
   SEQ ID NO. 4:
   H-Arg-Arg-Leu-Phe-[Cys-Tyr-Trp-Lys-Thr-Cys]-Thr-Trp-Arg-Asp-OH.

5. A peptide-drug conjugate targeting SSTR2,comprising a peptide targeting SSTR2 and a spacer conjugated to the peptide, wherein:
   the peptide targeting SSTR2 comprises the following sequence:

   Arg-X1-Phe-[Cys-Xaa-Trp-Lys-Thr-Cys]-Thr-X2-Arg-X3-NH$_2$/OH;

   wherein a disulfide bond is formed between the two Cys residues within the square brackets"[]";
   Xaa is selected from one or more of Tyr and Phe; X1 and X2 are selected from any amino acid residues; X3 is selected from any amino acid residues or is absent;
   the spacer has the following structure:
   X4-X6-X5,

   wherein X4 is absent or is a single bond, an acetyl group, or a polyether structure conjugated to the peptide;
   X5 is a polyether structure; X6 is an amino acid residue containing a reactive group,
   preferably selected from a group consisting of a carboxyl group, a hydroxyl group, an amino group, a thiol group, and an aldehyde group.

6. The peptide-drug conjugate targeting SSTR2 according to claim 5, wherein the polyether has a polyethylene glycol structure with 2 to 12 repeating units.

7. The peptide-drug conjugate targeting SSTR2 according to claim 5, wherein the amino acid residue containing a reactive group is selected from group consisting of a cysteine residue, lysine residue, ornithine residue, glutamic acid residue, aspartic acid residue, 6-azido-L-norleucine, 2-amino-5-hydroxypentanoic acid residue, 2-amino-5-ureidopentanoic acid residue, and 2-amino-5-cyanobenzoic acid residue.

8. The peptide-drug conjugate targeting SSTR2 according to claim 5, wherein the sequence is:
X0-Arg-X1-Phe-[Cys-Xaa-Trp-Lys-Thr-Cys]-Thr-X2-Arg-X3-NH$_2$/OH;
wherein X0 is selected from amino acid residues capable of reacting with an amino group or from other carboxyl-containing groups, and X0 is more preferably Arg or Gly.

9. The peptide-drug conjugate targeting SSTR2 according to claim 5, wherein the spacer further contains other amino acid residues, for example, the spacer may have the following structure:
X4'-X6-{Lys(X4-X6'-X5)}x-AAy-X5',

wherein AA is a random sequence of amino acid residues, y is an integer from 0 to 4, and x is an integer from 0 to 3, wherein X4' and X4 are identical or different, and independently is absent or is a single bond, an acetyl group, or a polyether structure conjugated to the peptide; X5' and X5 are identical or different, and each independently has a polyether structure;
X6 and X6' are identical or different, and each independently is an amino acid residue containing a reactive group.

10. The peptide-drug conjugate targeting SSTR2 according to claim 9, wherein the spacer is selected from a group consisting of: X6-(AEEA)r, PEGz-X6-PEGz, Ac-X6-PEGz, Ac-X6-(AEEA)r, Ac-X6-PEG6-{Lys(Ac-X6'-PEG6)}x-PEG6, and X6-Glu-Glu-Glu-AEEA, wherein r is an integer, preferably an integer from 0 to 6, more preferably an integer from 1 to 5, such as r = 2, 3, or 4.

11. The peptide-drug conjugate targeting SSTR2 according to claim 9, further comprising a linker for conjugating a active pharmaceutical ingredient, wherein the linker is conjugated to X6 and/or X6' residues of the spacer; preferably, the linker is selected from a group consisting of MC-Val-Ala-PABC, MC-Val-Cit-PABC, MC-Gly-Gly-Phe-Gly-NH-CH2, MCC, MC-betaglucuronide, MC-Glu-Val-Ala-PABC, and MC-Glu-Val-Cit-PABC.

12. The peptide-drug conjugate targeting SSTR2 according to claim 7, further comprising an active pharmaceutical ingredient, wherein the active pharmaceutical ingredient is conjugated to the linker; preferably, the active pharmaceutical ingredient is conjugated to the linker by one or more of condensation, esterification, etherification, and amidation reaction.

13. The peptide-drug conjugate according to claim 12, wherein the active pharmaceutical ingredient is selected from cytotoxic agents, preferably from a group consisting of: microtubule inhibitors (such as auristatins, maytansine derivatives, tubulysins, and cryptomycins), DNA-damaging drugs (such as pyrrolobenzodiazepines and indochloropyrrolobenzodiazepines, duocarmycin, camptothecin, and calicheamicin), amatoxins, apoptosis inducers, and camptothecins; more preferably, the cytotoxic agent is selected from the group consisting of MMAF, MMAE, PBD, DM1, DM4, Dxd, SN38, paclitaxel, docetaxel, and exatecan.

14. The peptide-drug conjugate targeting SSTR2 according to claim 12, wherein the peptide-drug conjugate is selected from

Drug 1:

Cys(MC-Val-Cit-PABC-MMAE)-AEEA-Arg-Arg-Leu-Phe-[Cys-Tyr-D-Trp-Lys-Thr-Cys]-Thr-Trp-Arg-Asp-NH$_2$

Drug 2:

PEG$_6$-Cys(MC-Val-Cit-PABC-MMAE)-PEG$_6$-Arg-Arg-Leu-Phe-[Cys-Tyr-D-Trp-Lys-Thr-Cys]-Thr-Trp-Arg-Asp-NH$_2$

Drug 3:

Ac-Cys(MC-Val-Cit-PABC-MMAE)-PEG$_6$-Arg-Arg-Leu-Phe-[Cys-Tyr-D-Trp-Lys-Thr-Cys]-Thr-Trp-Arg-Asp-NH$_2$

Drug 4:

Ac-Cys(MC-betaglucuronide-MMAE)-AEEA-AEEA-AEEA-Arg-Arg-Leu-Phe-[Cys-Tyr-D-Trp-Lys-Thr-Cys]-Thr-Trp-Arg-Asp-NH$_2$

Drug 5:

PEG$_6$-Cys(MC-Gly-Gly-Phe-Gly-NH-CH$_2$-Dxd)-PEG$_6$-Arg-Arg-Leu-Phe-[Cys-Tyr-D-Trp-Lys-Thr-Cys]-Thr-Trp-Arg-Asp-NH$_2$

Drug 6:

Ac-Cys(MC-Gly-Gly-Phe-Gly-NH-CH$_2$-Dxd)-PEG$_6$-Arg-Arg-Leu-Phe-[Cys-Tyr-D-Trp-Lys-Thr-Cys]-Thr-Trp-Arg-Asp-NH$_2$

Drug 7:

Ac-Cys(MC-Gly-Gly-Phe-Gly-NH-CH$_2$-Dxd)-PEG$_6$-Lys(Ac-Cys(MC-Gly-Gly-Phe-Gly-NH-CH$_2$-Dxd)-PEG$_6$)-Lys(Ac-Cys(MC-Gly-Gly-Phe-Gly-NH-CH$_2$-Dxd)-PEG$_6$)-PEG$_6$-Arg-Arg-Leu-Phe-[Cys-Tyr-D-Trp-Lys-Thr-Cys]-Thr-Trp-Arg-Asp-N H$_2$

Drug 8:

Ac-Cys(MC-Val-Cit-PABC-MMAE)-AEEA-AEEA-AEEA-Arg-Arg-Leu-Phe-[Cys-Phe-D-Trp-Lys-Thr-Cys]-Thr-Trp-Arg-Asp-NH$_2$

Drug 9:

Cys(MC-Val-Cit-PABC-MMAE)-Glu-Glu-Glu-AEEA-Arg-Arg-Leu-Phe-[Cys-Tyr      D-Trp-Lys-Thr-Cys]-Thr-Trp-Arg-Asp-NH$_2$

Drug 10:

Ac-Cys(MC-Val-Cit-PABC-MMAE)-AEEA-Arg-Arg-Leu-Phe-[Cys-Tyr-D-Trp-Lys-Thr-Cys]-Thr-Trp-Arg-Asp-NH$_2$.

15. The peptide-drug conjugate according to claim 12, wherein the peptide-drug conjugate contains a pharmaceutically acceptable (radioactive) isotope label or marker, or the active pharmaceutical ingredient is an isotope or marker, for example, one or more atoms of the peptide-drug conjugate are replaced by an atom having the same atomic number but an atomic mass or mass number different from that normally found in nature, or the active pharmaceutical ingredient is a metal chelating group capable of holding a radioactive isotope or marker, or certain functional groups in the peptide-drug conjugate are covalently substituted with related radioactive isotope- or marker-labeled functional groups.

16. The peptide-drug conjugate according to claim 15, wherein the isotope include: hydrogen isotopes such as $^2$H and $^3$H, carbon isotopes such as $^{11}$C, $^{13}$C and $^{14}$C, chlorine isotopes such as $^{36}$Cl, fluorine isotopes such as $^{18}$F, iodine isotopes such as $^{123}$I, $^{125}$I and $^{131}$I, nitrogen isotopes such as $^{13}$N and $^{15}$N, oxygen isotopes such as $^{15}$O, $^{17}$O and $^{18}$O, phosphorus isotopes such as $^{32}$P, sulfur isotopes such as $^{35}$S, copper isotopes such as $^{64}$Cu, gallium isotopes such as $^{67}$Ga or $^{68}$Ga, yttrium isotopes such as $^{90}$Y, lutetium isotopes such as $^{177}$Lu, and bismuth isotopes such as $^{213}$Bi.

17. Use of a peptide targeting SSTR2 or a peptide-drug conjugate targeting SSTR2, wherein the peptide-drug conjugate targeting SSTR2 contains a peptide targeting SSTR2 and a spacer conjugated to the peptide, or further contains a linker conjugated to the spacer, or further contains a payload conjugated to the linker;

wherein the peptide targeting SSTR2 or the peptide-drug conjugate targeting SSTR2 is used for preventing, inhibiting, or treating a disease with high expression of SSTR2, or for preparing a medicament for treating, inhibiting, or preventing a disease with high expression of SSTR2;
wherein the peptide targeting SSTR2 comprises the following sequence:
Arg-X1-Phe-[Cys-Xaa-Trp-Lys-Thr-Cys]-Thr-X2-Arg-X3-NH$_2$/OH;

wherein a disulfide bond is formed between the two Cys residues within the square brackets"[]";
Xaa is selected one or more from a group consisting of Tyr and Phe; X1 and X2 are selected from any amino acid residues; X3 is selected from any amino acid residues or is absent;
the spacer has the following structure: X4-X6-X5, wherein X4 is absent or is a single bond, an acetyl group, or a polyether structure conjugated to the peptide; X5 has a polyether structure; X6 is an amino acid residue containing a reactive group, preferably selected from a group consisting of carboxyl group, a hydroxyl group, an amino group, a thiol group, or an aldehyde group.

18. The use according to claim 17, wherein the disease is cancer, particularly preferably solid tumors originating from neuroendocrine cells, such as neuroendocrine tumors of the digestive system such as the stomach, intestines, and pancreas, and small cell lung cancer.

19. The use according to claim 17, wherein the sequence is:
X0-Arg-X1-Phe-[Cys-Xaa-Trp-Lys-Thr-Cys]-Thr-X2-Arg-X3-NH$_2$/OH;
wherein X0 is selected from amino acid residues capable of reacting with an amino group or from other carboxyl-containing groups, and X0 is more preferably Arg or Gly.

20. The use according to claim 17, wherein the spacer may have the following structure:
X4'-X6- {Lys(X4-X6'-X5)} x-AAy-X5',

wherein AA is a random sequence of amino acid residues, y is an integer from 0 to 4, and x is an integer from 0 to 3, wherein X4' and X4 are identical or different, and independently are absent or is a single bond, an acetyl group, or a polyether structure conjugated to the peptide; X5' and X5 are identical or different, and each independently is a polyether structure;
X6 and X6' are identical or different, and each independently is an amino acid residue containing a reactive group;
the linker is conjugated to X6 and/or X6' residues of the spacer.

# EP 4 772 530 A1

## INTERNATIONAL SEARCH REPORT

| | International application No. |
|---|---|
| | **PCT/CN2023/115938** |

| A. | CLASSIFICATION OF SUBJECT MATTER |
|---|---|
| | C07K7/08(2006.01)i; A61K47/64(2017.01)i; A61K31/7068(2006.01)i; A61P35/00(2006.01)i |

According to International Patent Classification (IPC) or to both national classification and IPC

| B. | FIELDS SEARCHED |
|---|---|

Minimum documentation searched (classification system followed by classification symbols)

IPC: C07K, A61K, A61P

Documentation searched other than minimum documentation to the extent that such documents are included in the fields searched

Electronic data base consulted during the international search (name of data base and, where practicable, search terms used)

CNABS; CNTXT; DWPI; WPABS; ENTXT; OETXT; VEN; CNKI; PubMed; WEB OF SCIENCE and keywords: 生长抑素, 生长抑素受体, SSTR2, SST, 肿瘤靶向肽, 肿瘤, 癌, tumor homing peptides, THPs, cancer, tumor; 中国专利生物序列检索系统, China Patent Biological Sequence Search System; Genbank和检索的序列: SEQ ID NO: 1, Genbank and searched sequence: SEQ ID NO: 1.

| C. | DOCUMENTS CONSIDERED TO BE RELEVANT |
|---|---|

| Category* | Citation of document, with indication, where appropriate, of the relevant passages | Relevant to claim No. |
|---|---|---|
| A | CN 114515339 A (DIVBIO PHARMACEUTICAL CO,. LTD.) 20 May 2022 (2022-05-20) see claims 1-5 | 1-20 (in part) |
| A | CN 115583989 A (YANTAI LANNACHENG BIOTECHNOLOGY CO., LTD.) 10 January 2023 (2023-01-10) see claims 1-11 | 1-20 (in part) |
| A | CN 113577284 A (SHENZHEN TYERCAN BIO-PHARM CO., LTD.) 02 November 2021 (2021-11-02) see claims 1-10 | 1-20 (in part) |
| A | WO 03102594 A1 (BAYER AG) 11 December 2003 (2003-12-11) see claims 1-26 | 1-20 (in part) |
| A | CN 115671305 A (THE SECOND HOSPITAL OF TIANJIN MEDICAL UNIVERSITY) 03 February 2023 (2023-02-03) see claims 1-3 | 1-20 (in part) |

| ✓ Further documents are listed in the continuation of Box C. | ✓ See patent family annex. |
|---|---|

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **15 May 2024** | **25 May 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2023/115938** |

| C. | DOCUMENTS CONSIDERED TO BE RELEVANT | |
| --- | --- | --- |
| Category* | Citation of document, with indication, where appropriate, of the relevant passages | Relevant to claim No. |
| A | US 2007066516 A1 (SRINIVASAN ANANTH) 22 March 2007 (2007-03-22)<br>see claims 1-27 | 1-20 (in part) |

Form PCT/ISA/210 (second sheet) (July 2022)

**INTERNATIONAL SEARCH REPORT**

International application No.

**PCT/CN2023/115938**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

| International application No. |
| --- |
| **PCT/CN2023/115938** |

**Box No. II   Observations where certain claims were found unsearchable (Continuation of item 2 of first sheet)**

This international search report has not been established in respect of certain claims under Article 17(2)(a) for the following reasons:

1. ☑  Claims Nos.: **17-20**
   because they relate to subject matter not required to be searched by this Authority, namely:

   Part of the technical solutions of claims 17-20 "a peptide targeting SSTR2 or a peptide-drug conjugate targeting SSTR2 being used for preventing, inhibiting or treating diseases with high expression of SSTR2" belongs to PCT Rule 39.1(iv) – methods for treatment of the human or animal body by surgery or therapy. With regard to said claims, the reasonably expected subject matter is: the use of a peptide targeting SSTR2 or a peptide-drug conjugate targeting SSTR2 in the preparation of a drug for treating, inhibiting or preventing diseases with high expression of SSTR2, characterized in that the peptide-drug conjugate targeting SSTR2 contains a peptide targeting SSTR2 and a spacer connected to the peptide, and may also contain a linker connected to the spacer, or further contains an active drug connected to the linker.

2. ☐  Claims Nos.:
   because they relate to parts of the international application that do not comply with the prescribed requirements to such an extent that no meaningful international search can be carried out, specifically:

3. ☐  Claims Nos.:
   because they are dependent claims and are not drafted in accordance with the second and third sentences of Rule 6.4(a).

**Box No. III   Observations where unity of invention is lacking (Continuation of item 3 of first sheet)**

This International Searching Authority found multiple inventions in this international application, as follows:

Invention 1: claims 1-20 (in part) relate to a peptide targeting SSTR2, which peptide has the following sequence: Arg-Arg-Leu-Phe-[Cys-Tyr-Trp-Lys-Thr-Cys]-Thr-Trp-Arg-Asp-$NH_2$; and a corresponding peptide-drug conjugate targeting SSTR2 and a pharmaceutical use;

invention 2: claims 1-20 (in part) relate to a peptide targeting SSTR2, which peptide has the following sequence: Arg-Arg-Leu-Phe-[Cys-Phe-Trp-Lys-Thr-Cys]-Thr-Trp-Arg-Asp-$NH_2$; and a corresponding peptide-drug conjugate targeting SSTR2 and a pharmaceutical use;

invention 3: claims 1-20 (in part) relate to a peptide targeting SSTR2, which peptide has the following sequence: Arg-Arg-Arg-Phe-[Cys-Tyr-Trp-Lys-Thr-Cys]-Thr-Trp-Arg-Asp-$NH_2$; and a corresponding peptide-drug conjugate targeting SSTR2 and a pharmaceutical use;

invention 4: claims 1-20 (in part) relate to a peptide targeting SSTR2, which peptide has the following sequence: Arg-Arg-Arg-Phe-[Cys-Phe-Trp-Lys-Thr-Cys]-Thr-Trp-Arg-Asp-$NH_2$; and a corresponding peptide-drug conjugate targeting SSTR2 and a pharmaceutical use;

invention 5: claims 1-20 (in part) relate to a peptide targeting SSTR2, which peptide has the following sequence: Arg-Arg-Leu-Phe-[Cys-Tyr-Trp-Lys-Thr-Cys]-Thr-Asp-Arg-Asp-$NH_2$; and a corresponding peptide-drug conjugate targeting SSTR2 and a pharmaceutical use;

invention 6: claims 1-20 (in part) relate to a peptide targeting SSTR2, which peptide has the following sequence: Arg-Arg-Leu-Phe-[Cys-Phe-Trp-Lys-Thr-Cys]-Thr-Asp-Arg-Asp-$NH_2$; and a corresponding peptide-drug conjugate targeting SSTR2 and a pharmaceutical use;

invention 7: claims 1-20 (in part) relate to a peptide targeting SSTR2, which peptide has the following sequence: Arg-Arg-Arg-Phe-[Cys-Tyr-Trp-Lys-Thr-Cys]-Thr-Asp-Arg-Asp-$NH_2$; and a corresponding peptide-drug conjugate targeting SSTR2 and a pharmaceutical use;

invention 8: claims 1-20 (in part) relate to a peptide targeting SSTR2, which peptide has the following sequence: Arg-Arg-Arg-Phe-[Cys-Phe-Trp-Lys-Thr-Cys]-Thr-Asp-Arg-Asp-$NH_2$; and a corresponding peptide-drug conjugate targeting SSTR2 and a pharmaceutical use;

Form PCT/ISA/210 (continuation of first sheet) (July 2022)

## INTERNATIONAL SEARCH REPORT

| International application No. |
|---|
| **PCT/CN2023/115938** |

**Box No. III    Observations where unity of invention is lacking (Continuation of item 3 of first sheet)**

invention 9: claims 1-20 (in part) relate to a peptide targeting SSTR2, which peptide has the following sequence: Arg-Leu-Phe-[Cys-Tyr-Trp-Lys-Thr-Cys]-Thr-Trp-Arg-Asp-NH$_2$; and a corresponding peptide-drug conjugate targeting SSTR2 and a pharmaceutical use;

invention 10: claims 1-20 (in part) relate to a peptide targeting SSTR2, which peptide has the following sequence: Arg-Leu-Phe-[Cys-Phe-Trp-Lys-Thr-Cys]-Thr-Trp-Arg-Asp-NH$_2$; and a corresponding peptide-drug conjugate targeting SSTR2 and a pharmaceutical use;

invention 11: claims 1-20 (in part) relate to a peptide targeting SSTR2, which peptide has the following sequence: Arg-Arg-Phe-[Cys-Tyr-Trp-Lys-Thr-Cys]-Thr-Trp-Arg-Asp-NH$_2$; and a corresponding peptide-drug conjugate targeting SSTR2 and a pharmaceutical use;

invention 12: claims 1-20 (in part) relate to a peptide targeting SSTR2, which peptide has the following sequence: Arg-Arg-Phe-[Cys-Phe-Trp-Lys-Thr-Cys]-Thr-Trp-Arg-Asp-NH$_2$; and a corresponding peptide-drug conjugate targeting SSTR2 and a pharmaceutical use;

invention 13: claims 1-20 (in part) relate to a peptide targeting SSTR2, which peptide has the following sequence: Arg-Leu-Phe-[Cys-Tyr-Trp-Lys-Thr-Cys]-Thr-Asp-Arg-Asp-NH$_2$; and a corresponding peptide-drug conjugate targeting SSTR2 and a pharmaceutical use;

invention 14: claims 1-20 (in part) relate to a peptide targeting SSTR2, which peptide has the following sequence: Arg-Leu-Phe-[Cys-Phe-Trp-Lys-Thr-Cys]-Thr-Asp-Arg-Asp-NH$_2$; and a corresponding peptide-drug conjugate targeting SSTR2 and a pharmaceutical use;

invention 15: claims 1-20 (in part) relate to a peptide targeting SSTR2, which peptide has the following sequence: Arg-Arg-Phe-[Cys-Tyr-Trp-Lys-Thr-Cys]-Thr-Asp-Arg-Asp-NH$_2$; and a corresponding peptide-drug conjugate targeting SSTR2 and a pharmaceutical use;

invention 16: claims 1-20 (in part) relate to a peptide targeting SSTR2, which peptide has the following sequence: Arg-Arg-Phe-[Cys-Phe-Trp-Lys-Thr-Cys]-Thr-Asp-Arg-Asp-NH$_2$; and a corresponding peptide-drug conjugate targeting SSTR2 and a pharmaceutical use;

invention 17: claims 1-20 (in part) relate to a peptide targeting SSTR2, which peptide has the following sequence: Gly-Arg-Leu-Phe-[Cys-Tyr-Trp-Lys-Thr-Cys]-Thr-Trp-Arg-Asp-NH$_2$; and a corresponding peptide-drug conjugate targeting SSTR2 and a pharmaceutical use;

invention 18: claims 1-20 (in part) relate to a peptide targeting SSTR2, which peptide has the following sequence: Gly-Arg-Leu-Phe-[Cys-Phe-Trp-Lys-Thr-Cys]-Thr-Trp-Arg-Asp-NH$_2$; and a corresponding peptide-drug conjugate targeting SSTR2 and a pharmaceutical use;

invention 19: claims 1-20 (in part) relate to a peptide targeting SSTR2, which peptide has the following sequence: Gly-Arg-Arg-Phe-[Cys-Tyr-Trp-Lys-Thr-Cys]-Thr-Trp-Arg-Asp-NH$_2$; and a corresponding peptide-drug conjugate targeting SSTR2 and a pharmaceutical use;

invention 20: claims 1-20 (in part) relate to a peptide targeting SSTR2, which peptide has the following sequence: Gly-Arg-Arg-Phe-[Cys-Phe-Trp-Lys-Thr-Cys]-Thr-Trp-Arg-Asp-NH$_2$; and a corresponding peptide-drug conjugate targeting SSTR2 and a pharmaceutical use;

invention 21: claims 1-2 (in part) and 5-20 (in part) relate to a peptide targeting SSTR2, which peptide has the following sequence: Gly-Arg-Leu-Phe-[Cys-Tyr-Trp-Lys-Thr-Cys]-Thr-Asp-Arg-Asp-NH$_2$; and a corresponding peptide-drug conjugate targeting SSTR2 and a pharmaceutical use;

invention 22: claims 1-20 (in part) relate to a peptide targeting SSTR2, which peptide has the following sequence: Gly-Arg-Leu-Phe-[Cys-Phe-Trp-Lys-Thr-Cys]-Thr-Asp-Arg-Asp-NH$_2$; and a corresponding peptide-drug conjugate targeting SSTR2 and a pharmaceutical use;

invention 23: claims 1-20 (in part) relate to a peptide targeting SSTR2, which peptide has the following sequence: Gly-Arg-Arg-Phe-[Cys-Tyr-Trp-Lys-Thr-Cys]-Thr-Asp-Arg-Asp-NH$_2$; and a corresponding peptide-drug conjugate targeting SSTR2 and a pharmaceutical use;

invention 24: claims 1-20 (in part) relate to a peptide targeting SSTR2, which peptide has the following sequence: Gly-Arg-Arg-Phe-[Cys-Phe-Trp-Lys-Thr-Cys]-Thr-Asp-Arg-Asp-NH$_2$; and a corresponding

peptide-drug conjugate targeting SSTR2 and pharmaceutical use;

Form PCT/ISA/210 (continuation of first sheet) (July 2022)

## INTERNATIONAL SEARCH REPORT

International application No.

**PCT/CN2023/115938**

| Box No. III     Observations where unity of invention is lacking (Continuation of item 3 of first sheet) |
| --- |

inventions 25-40: claims 1-20 (in part) relate to peptides targeting SSTR2, which peptides have the following sequence:

Arg-X1-Phe-[Cys-Xaa-Trp-Lys-Thr-Cys]-Thr-X2-Arg-X3-NH$_2$/OH, or X0-Arg-X1-Phe-[Cys-Xaa-Trp-Lys-Thr-Cys]-Thr-X2-Arg-X3-NH$_2$/OH, wherein Xaa is selected from two of Tyr and Phe, X1 is Arg or Leu, X2 is Trp or Asp, X3 is Asp, and X0 is Arg or Gly; and corresponding peptide-drug conjugates targeting SSTR2 and pharmaceutical uses; and

invention 41: claims 1-20 (in part) relate to a peptide targeting SSTR2, which peptide has the following sequence:

Arg-X1-Phe-[Cys-Xaa-Trp-Lys-Thr-Cys]-Thr-X2-Arg-X3-NH$_2$/OH, or X0-Arg-X1-Phe-[Cys-Xaa-Trp-Lys-Thr-Cys]-Thr-X2-Arg-X3-NH$_2$/OH, wherein Xaa is selected from one or two of Tyr and Phe, X1 is an amino acid other than Arg or Leu, X2 is an amino acid other than Trp or Asp, and X3 is an amino acid other than Asp or does not exist; and a corresponding peptide-drug conjugate targeting SSTR2 and a pharmaceutical use.

The same or corresponding technical feature of the 41 inventions is the following sequence "Lys-Thr-Cys-Thr". This technical feature has been disclosed in the prior art (see, for example, CN 114515339 A, publication date: 20 May 2022, a polypeptide-drug conjugate for an somatostatin receptor comprising the above amino acid sequence, which is disclosed therein). Therefore, the same or corresponding technical feature among inventions 1-41 does not defines a contribution which the inventions make over the prior art, and cannot be considered to be a special technical feature (PCT Rule 13.2). Therefore, the present application does not comply with the requirement of unity of invention (PCT Rule 13.1).

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **Invention 1: claims 1-20 (in part)**

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

**INTERNATIONAL SEARCH REPORT**
Information on patent family members

International application No.

**PCT/CN2023/115938**

| Patent document cited in search report | | | Publication date (day/month/year) | Patent family member(s) | | | Publication date (day/month/year) |
|---|---|---|---|---|---|---|---|
| CN | 114515339 | A | 20 May 2022 | None | | | |
| CN | 115583989 | A | 10 January 2023 | None | | | |
| CN | 113577284 | A | 02 November 2021 | None | | | |
| WO | 03102594 | A1 | 11 December 2003 | EP | 1371987 | A1 | 17 December 2003 |
| | | | | AU | 2003240736 | A1 | 19 December 2003 |
| CN | 115671305 | A | 03 February 2023 | None | | | |
| US | 2007066516 | A1 | 22 March 2007 | EP | 1717247 | A1 | 02 November 2006 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **NAIR et al.** *J Immunol*, 2003, vol. 170 (3), 1362-1373